(19)

Europäisches
Patentamt
European
Patent Office
Office européen
des brevets

(11) **EP 1 627 618 B1**

(12) **EUROPEAN PATENT SPECIFICATION**

(45) Date of publication and mention
of the grant of the patent:
**24.02.2010 Bulletin 2010/08**

(51) Int Cl.:
*A61F 13/15* (2006.01)   *D04H 3/00* (2006.01)

(21) Application number: **05018160.1**

(22) Date of filing: **22.08.2005**

(54) **Process of producing an absorbent member**

Verfahren zur Herstellung eines absorbierenden Artikels

Procede pour la production d'un article absorbant

(84) Designated Contracting States:
**DE FR GB SE**

(30) Priority: **20.08.2004   JP 2004240670**
**20.12.2004   JP 2004368393**
**21.12.2004   JP 2004368618**
**23.03.2005   JP 2005082913**

(43) Date of publication of application:
**22.02.2006   Bulletin 2006/08**

(73) Proprietor: **KAO CORPORATION**
**Chuo-ku,**
**Tokyo (JP)**

(72) Inventors:
 • **Kasai, Takao,**
 **Kao Corporation**
 **Haga-gun**
 **Tochigi (JP)**

 • **Kouta, Takuya,**
 **Kao Corporation**
 **Haga-gun**
 **Tochigi (JP)**
 • **Niinomi, Masahiko,**
 **Kao Corporation**
 **Haga-gun**
 **Tochigi (JP)**

(74) Representative: **Vossius & Partner**
 **Siebertstraße 4**
 **81675 München (DE)**

(56) References cited:
 **EP-A- 1 323 400        EP-A- 1 417 946**
 **US-A1- 2003 134 559**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a process of producing an absorbent member used in an absorbent article, particularly a stretchable absorbent member.

An absorbent member for absorbent articles using an opened tow of continuous filaments is known. Included is an absorbent member comprising a crimped cellulose acetate fiber tow layer and a ground pulp layer accumulated on one side of the tow layer, the two layers being united by pressing in the thickness direction (see JP-A-57-160457). The absorbent member is described as having improved body fluid distribution. However, because cellulose acetate fiber is inferior in water absorption to pulp, a large quantity of ground pulp should be used in combination to secure increased absorption capacity, which results in increased thickness and deteriorated wearing comfort of the absorbent article

WO2001/34082 proposes an absorbent core composed of an upper layer, a lower layer, and an absorbent layer interposed therebetween. The absorbent layer includes a superabsorbent polymer sprinkled layer on which of a fiber layer made of cellulose acetate fiber tow is disposed. The superabsorbent polymer has a part thereof adhesively bonded to the lower layer and another part thereof entering the fiber tow layer. Although part of the superabsorbent polymer enters the fiber tow layer, the most part of the superabsorbent polymer is bonded to the lower layer. In other words, the fiber tow layer and the superabsorbent polymer sprinkled layer are independent of each other. It follows that the structure of the absorbent core is destroyed easily when deformed during wear by the wearer's movement.

Among known absorbent layers having a tow of continuous filaments is the one disclosed in JP-A-2001-276125, in which the tow extends in the thickness direction of the absorbent layer. The publication says that a body fluid moves downward through the interstices of the tow and draws away from the wearer's skin and is thus prevented from causing overhydration or rash. In order for the absorbent layer to have such a structure, the tow should have some length so that the absorbent layer is of necessity thick.

None of the above-described absorbent members have stretchability. While worn, an absorbent article having such a non-stretchable absorbent member, e.g., a disposable diaper, is incapable of sufficiently following the movement of a wearer's body and easily bunches up or loses a good fit.

Apart from the foregoing techniques, Applicant previously proposed in JP-A-2004-159786 an absorbent article having an absorbent member containing crimped fiber. According to the disclosure, the absorbent member has less bunching, increased absorptivity, and improved comfort and fit while worn. Staple fiber is usually used as the crimped fiber.

In a first aspect an absorbent article is described having an absorbent member containing a web of hydrophilic long fibers. The long fibers have a crimp percentage of 40% to 90% and are highly oriented in the planar direction of the absorbent member. The web has a superabsorbent polymer which is embeddedly in the web.

In a second aspect a stretchable absorbent member is described having a web of hydrophilic long fibers, a superabsorbent polymer which is embeddedly supported in the web, and a liquid permeable sheet which wraps the web. The hydrophilic long fibers have a crimp percentage of 40% to 90%. The web has a high degree of orientation in the planar direction of the absorbent member. The liquid permeable sheet is extensible to ensure the stretchability of the absorbent member.

In a third aspect an absorbent article is described having elastic regions which extend along at least one of the longitudinal direction and the width direction and are located along at least one of a pair of lateral sides and a pair of longitudinal ends of the absorbent article. The absorbent article has an absorbent member which is disposed in the elastic regions. The absorbent member is stretchable and has a web containing hydrophilic crimped long fibers with a high degree of orientation in one direction. The web has a superabsorbent polymer which is embeddedly supported in the web.

Furthermore, an absorbent member is described having webs of hydrophilic long fibers having a crimp percentage of 40% to 90% and a layer of a superabsorbent polymer sprinkled between the webs. Part of the superabsorbent polymer is embeddedly supported in the web. The webs are bonded together in points.

The present invention according to any of claims 1 to 5 provides a process of producing an absorbent member having a web of crimped long fibers, the web containing a superabsorbent polymer. The process includes the steps of opening a web in a longitudinally stretched state under tension and feeding the superabsorbent polymer to the web in a state under reduced tension.

Fig. 1 schematically illustrates an embodiment of an absorbent member in the absorbent article.
Fig. 2(a) and Fig. 2(b) each illustrate another embodiment of the absorbent member in an absorbent article.
Fig. 3 schematically illustrates another embodiment of the absorbent member in an absorbent article.
Fig. 4 schematically illustrates another embodiment of the absorbent member in an absorbent article.
Fig. 5 schematically illustrates apparatus preferably used to produce the absorbent member in an absorbent article.
Fig. 6(a), Fig. 6(b), and Fig. 6(c) each illustrate another embodiment of the absorbent member in an absorbent article.
Fig. 7(a), Fig. 7(b), Fig. 7(c), and Fig. 7(d) each schematically illustrate another embodiment of the absorbent member in an absorbent article.

Fig. 8(a) and Fig. 8(b) each schematically illustrate another embodiment of the absorbent member in an absorbent article.

Fig. 9 schematically illustrates another embodiment of the absorbent member in an absorbent article.

Fig. 10 schematically illustrates another embodiment of the absorbent member in an absorbent article.

Fig. 11 schematically illustrates another embodiment of the absorbent member in an absorbent article.

Fig. 12 schematically illustrates another embodiment of the absorbent member in an absorbent article.

Fig. 13 is a schematic fragmentary cross-sectional view of an embodiment of an absorbent article.

Fig. 14 is a schematic fragmentary cross-sectional view of another embodiment of the absorbent article corresponding to Fig. 13.

Fig. 15 is a perspective of an embodiment of the extensible absorbent member according to the second aspect.

Fig. 16 is a perspective of the absorbent member shown in Fig. 15 in its stretched state.

Fig. 17 is a fragmentary cross-section of another embodiment of the extensible absorbent member according to the second aspect.

Fig. 18 is a fragmentary cross-section of another embodiment of the extensible absorbent member according to the second aspect.

Fig. 19 is a perspective of a disposable diaper according to the third aspect in which the extensible absorbent member of the second aspect is used.

Fig. 20 is a cross-section of Fig. 19, taken along line a-a.

Fig. 21 is a cross-section of Fig. 19, taken along line b-b.

Fig. 22 is a cross-section of Fig. 19 in its another embodiment, taken along line a-a.

Fig. 23 is a graph showing the relation between crimp percentage and superabsorbent polymer supporting ability of a long fiber web.

Fig. 24 is a graph showing the relation between particle shape and supportability of a superabsorbent polymer.

Fig. 25 is a graph representing the relation between diameter of long fiber and supportability of superabsorbent polymer.

Fig. 26 schematically illustrates the pattern of cutting slits in Examples.

The present invention will be described based on its preferred embodiments with reference to the accompanying drawings. The absorbent article described is designed primarily to absorb and retain body fluids including urine and menstrual blood. The absorbent article described typically includes, but is not limited to, disposable diapers, sanitary napkins, and incontinence pads. Also included are any other articles used to absorb fluids excreted from a human body.

The absorbent article described typically has a topsheet, a backsheet, and a liquid retentive absorbent member interposed between the topsheet and the backsheet. The topsheet and the backsheet can be of any material commonly used in the art. Useful topsheets are liquid permeable sheets made of hydrophilized nonwoven fabrics or perforated films. Useful backsheets are liquid impermeable or water repellent sheets such as a thermoplastic resin film and laminates of the film and nonwoven fabric. The backsheet may have water vapor permeability. The absorbent article may have other members configured for specific purposes of specific articles as well known to those skilled in the art. For example, an absorbent article for applications such as a disposable diaper or a sanitary napkin may have one or more pairs of standing cuffs on both lateral sides of the topsheet.

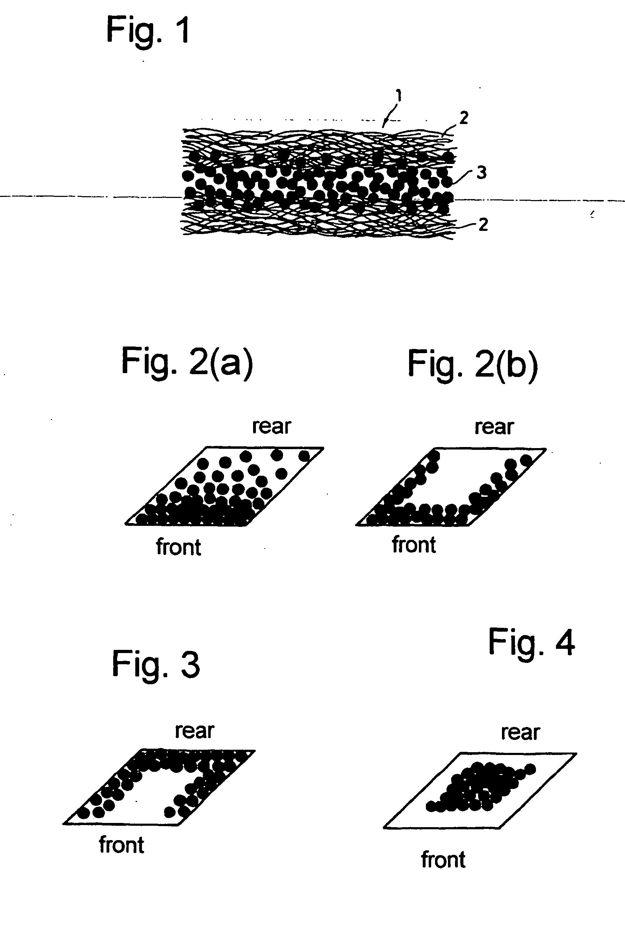

Fig. 1 is a schematic illustration of an embodiment of the absorbent member according to the first aspect. The absorbent member 1 of this embodiment is **characterized in that** it has sufficient absorption capacity and yet is thin and light weight. The thus characterized absorbent member 1 has a web containing long fibers (simply, the web 2), and a superabsorbent polymer sprinkled layer 3 (simply, the polymer layer 3). In more detail, the absorbent member 1 has at least two webs 2, and the polymer layers 3 disposed between the webs 2. The number of the webs 2 is not particularly limited and selected properly according to the intended use of the article. Considering that the absorbent member 1 is characterized by thinness and light weight, there is a natural limit to that number.

The long fibers are hydrophilic. Hydrophilic long fibers that can be used in the present invention include those which are essentially hydrophilic and those which are not essentially hydrophilic but rendered hydrophilic through a hydrophilization treatment. Those which are essentially hydrophilic are preferred. Cellulose acetate or rayon long fibers are more preferred. Cellulose acetate long fibers are particularly preferred for bulk retention even when wetted. Cellulose acetate is preferably cellulose triacetate or cellulose diacetate.

The long fibers are crimped fibers having a crimp percentage (specified in JIS L0208) of 40% to 90%, preferably 50% to 80%. By making a web from crimped long fibers, it is easy for a large quantity of a superabsorbent polymer to be embeddedly supported by the web stably. If a large amount of a superabsorbent polymer is supported between webs made solely of long fibers having no crimp or a small degree of crimping, the superabsorbent polymer tends to move extremely or fall off. In using long fibers of too high degree of crimping, on the other hand, it is not easy for a superabsorbent polymer to enter between the long fibers, and when the amount of the superabsorbent polymer is large, the polymer is liable to move extremely or fall off. The above-recited percentage of crimp is particularly suited as a structural material of an absorbent member with a flat, non-wavy surface. The percentage of crimp which is outside the above-recited range can also be used, if the absorbent member has a wavy surface which is prepared by, for example, embossing. The percentage of crimp of the absorbent member with a wavy surface is measured by the following procedure. The absorbent member is stretched with a tension as low as the wavy surface changes to a flat surface. Under this state, the percentage of crimp is measured in a usual manner. The means for crimping the long fibers is not particularly limited. The crimp may be either two-dimensional or three-dimensional. The percentage of crimp (or crimp percentage) is defined to be a percentage of a difference between the length A of a crimped fiber in its straightened state and the natural length B of the crimped fiber as crimped to the length A, being calculated from equation:

$$\text{Percentage of crimp (\%)} = (A-B)/A \times 100$$

The natural length of the crimped fiber is the length of the straight line connecting the two ends of a long fiber in its natural state. The term "natural state" means a state of a long fiber hanging under its own weight with its one end fixed to a horizontal plate. The term "straightened state" means a state of a long fiber stretched out to its full length under a minimum load.

The number of crimps of the long fibers having the recited percentage of crimp is preferably 2 to 25, more preferably 4 to 20, even more preferably 10 to 20, per centimeter.

The term "embeddedly supported" is intended to indicate the state in which the superabsorbent polymer enters the interfiber spaces formed by the crimped long fibers and therefore hardly moves extremely or falls off with the wearer's movement. This state is the results of the long fibers' entangling with or catching on the superabsorbent polymer or the polymer's attaching to the long fibers by its own stickiness. The spaces among the long fibers are secured even when externally stressed because they are deformable and also because the stress is absorbed by the whole long fibers. The superabsorbent polymer has part thereof embedded in the webs 2. Under some conditions of making the absorbent member 1, almost the whole of the superabsorbent polymer is uniformly embedded into the webs 2. Evaluation of the embedded state of the superabsorbent polymer will be described later.

The diameter of the long fibers is related to the properties of supporting the superabsorbent polymer. The weight per unit area of the web being equal, satisfactory polymer supporting properties can be obtained by using long fibers having a diameter of 1.0 to 7.8 dtex, preferably 1.7 to 5.6 dtex. The terminology "long fiber" as used throughout the description means a fiber having a mean fiber length preferably of 70 mm or longer, more preferably 80 mm or longer, even more preferably 100 mm or longer, as measured by the mean fiber length measurement method (method C) specified in JIS L1015. In case where the length of a web per se is shorter than 100 mm, "long fiber" is defined as follows. When preferably at least 50%, more preferably 70% or more, even more preferably 80% or more, of the fibers making up the web extend over the total length of the web, the fibers of the web are regarded as long fibers. The long fibers used in the present invention are those generally termed "continuous filaments". A bundle of continuous filaments is generally termed "a tow". Accordingly, the terminology "long fiber" as used herein shall include a continuous filament. The expression "a web having long fibers oriented" or its equivalent shall include a bundle of long fibers, namely, a tow, as a raw material for making a web and a tow layer of continuous filaments.

The superabsorbent polymer used in the art is usually particulate. A fibrous superabsorbent polymer is also useful. A particulate superabsorbent polymer which is irregular in shape or blocky or rodlike can be embeddedly supported in a web in an amount one to ten times the weight of the web. A particulate superabsorbent polymer which is spherical particles or agglomerates of spherical particles can be embeddedly supported in a web in an amount one to five times the weight of the web. Where both high absorption and thinness are demanded, the former type of polymers is preferably used. Where weight is put on hand (reduction of crispness characteristic of a superabsorbent polymer), it is desirable to use the latter type of the polymers. Blocky particles or agglomerates of spherical particles are particularly preferred for increasing the amount of the superabsorbent polymer embeddedly supported.

A superabsorbent polymer is sprinkled in a layer between the two webs 2. Part of the superabsorbent polymer is embedded in the web 2. Under some conditions of making the absorbent member 1, almost the whole of the superabsorbent polymer is uniformly embedded in the webs 2. The term "uniform" or "uniformly" is used not only when the superabsorbent polymer is distributed completely uniformly in the thickness or width direction of the absorbent member 1 but also when the variation in weight of the sprinkled superabsorbent polymer per unit area among different parts of the absorbent member 1 is such that the maximum is within double the minimum. Such variation is attributed to a phenomenon infrequently faced in the manufacture of an absorbent article that the superabsorbent polymer is fed in excess to partly sprinkle an extremely large amount of the polymer. That is, under the term "uniform" is included an unavoidable non-uniform distribution, but an intentional variation in amount of the superabsorbent polymer is not included under the term. The term "localized", which will be described later, means a state which is beyond the above-defined uniform distribution of the superabsorbent polymer, and includes an intentional variation in amount of the superabsorbent polymer.

Since the long fibers have crimp as stated, they furnish a great number of spaces capable of retaining particles, in which spaces the superabsorbent polymer is retained. Therefore, a large quantity of the superabsorbent polymer sprinkled on the long fiber web hardly moves extremely or falls off. Furthermore the structure of the absorbent member 1 is hardly destroyed by active movement of a wearer. The percentage of crimp and the amount of the long fibers are selected appropriately depending on the kind of the superabsorbent polymer used.

Supportability of the superabsorbent polymer in a long fiber web relates to the network structure formed by the web and the physical properties of the polymer. From the aspect of the network structure, the polymer supportability is achieved by the network with controlled three-dimensional regularity in terms of crimp percentage, fineness, density, etc. Because the fibers constituting the web are not bonded to each other, the mesh size of the network structure is changeable to an extent enough to hold and retain the superabsorbent polymer. Compared with a fiber aggregate having fibers bonded at the intersections such as nonwoven fabric, the web having a changeable network mesh size is highly capable of supporting the polymer. The network mesh size is controllable by, for example, (a) sprinkling the superabsorbent polymer on the web with tension given to the web and then releasing the web from the tension or (b) sprinkling the superabsorbent polymer on the web under controlled tension so as to have a specific crimp percentage and further applying tension or pressure to the web.

The physical properties of the superabsorbent polymer which relate to the supportability include particle size distribution, particle size, bulk density, surface conditions, internal frictional coefficient, fluidity, dispersibility, water content, electrostatic properties, stickiness, and agglomerating tendency. Of these properties the particle size distribution and the particle size are closely related to the network structure of the web. The supportability of the polymer is also affected by the number of collisions of the sprinkled polymer against the long fibers, which take place in the absorbent member due to the foreign force or vibration caused by the movement of a wearer. The larger the number of collisions, the less the supportability of the polymer. The number of collisions is influenced by the fluidity of the polymer due to the promoted sieving of the polymer by the network made of the long fibers. The higher the fluidity, the larger the number of collisions. The polymer with high fluidity that has once bounced off the web running at a high peed easily moves thereafter on the web and is hardly supported in the web.

Comparison is made between blocky polymer particles and agglomerates of spherical particles. Because the blocky polymer particles are more fluid than the agglomerates of spherical particles, the former exhibits higher supportability than the latter. Moreover, because the agglomerates of spherical particles have a smoother surface, they are less frictional or scratchy on the fibers than the blocky polymer. From this viewpoint, too, blocky polymer particles are more supportable than the agglomerates of spherical particles.

The webs of crimped long fibers having the superabsorbent polymer sandwiched therebetween may be subjected to various post-treatments to make it sure that the superabsorbent polymer be supported more efficiently. Such post-treatments include (1) wrapping the whole laminate (the webs and the layer of the superabsorbent polymer) in a sheet such as paper or nonwoven fabric or superposing the sheet on the laminate, (2) superposing a fluff pulp airlaid layer containing or not containing the superabsorbent polymer on the whole laminate, or (3) fixing the structure of the laminate with an adhesive or by heat application or ultrasonication.

The absorbent member 1 having the above-described structure is thin and light weight. The absorbent member 1 can have an appropriate thickness and weight according to the intended use of the absorbent article using the same. For applications to disposable diapers for infants, for instance, each of the webs 2 preferably has a weight of 5 to 200 $g/m^2$, more preferably 10 to 100 $g/m^2$. In this case, the two or more webs 2 may be the same or different in weight. The amount of the superabsorbent polymer in the absorbent member 1 is preferably 50 to 500 $g/m^2$, more preferably 100 to 300 $g/m^2$. Two or more polymer layers 3, if any, do no need to have the same weight per unit area.

For application to sanitary napkins, each of the webs 2 preferably has a weight of 5 to 100 $g/m^2$, more preferably 10 to 50 $g/m^2$, and the amount of the superabsorbent polymer is preferably 10 to 200 $g/m^2$, more preferably 15 to 100 $g/m^2$. For application to incontinence pads, each of the webs 2 preferably has a weight of 5 to 200 $g/m^2$, more preferably 10 to 100 $g/m^2$, and the amount of the superabsorbent polymer is preferably 10 to 500 $g/m^2$, more preferably 15 to 350 $g/m^2$.

Where the weight per unit area of the polymer layer 3 is large relative to that of each of the webs 2, the web 2 holds the superabsorbent polymer through its whole thickness. As a result, neighboring polymer layers 3 or neighboring webs 2 are not seemingly distinguishable, and the absorbent member 1 has the superabsorbent polymer distributed throughout the thickness thereof. In brief, the absorbent member according to the present invention includes in its scope an embodiment having a matrix of a hydrophilic long fiber web and a superabsorbent polymer dispersed uniformly in the thickness direction of the matrix.

The absorbent member 1 can retain a large quantity of the superabsorbent polymer since the polymer can be held stably in the spaces formed by the crimped long fibers. A conventional absorbent member could retain a large quantity of the superabsorbent polymer by increasing the amount of a fibrous material, which results in increases in weight and thickness of the absorbent member. In contrast, the present invention makes it possible to increase the amount of the superabsorbent polymer relative to the amount of the fibrous material. Specifically, the absorbent member 1 preferably contains the superabsorbent polymer in an amount equal to or larger than, more preferably twice or more as much as, even more preferably three times or more as much as, the weight of the long fibers. Such high polymer supportability has accomplished reduction in thickness and weight of the absorbent member 1. The maximum amount of the superabsorbent polymer with respect to the long fibers is decided in connection with prevention of extreme movement or fall-off of the polymer. While depending on the degree of crimping of the long fibers, the superabsorbent polymer hardly moves extremely or falls off against active wearer's movement as long as the weight of the superabsorbent polymer is not more than about ten times the weight of the long fibers.

The following method is used to determine how stably a superabsorbent polymer is embeddedly supported. A web measuring 100 mm in with and 200 mm in length is prepared. The web is cut into halves along the lateral centerline to obtain a 100 mm x 100 mm specimen. The half (specimen) is shaken 20 times at an amplitude of 5 cm at a rate of one shake per second with the cut area down. The polymer fallen from the cut area is weighed. When the rate of the fallen polymer is within 25%, preferably within 20%, more preferably within 10%, to the total polymer weight that has existed in the specimen, it is safe to say that the polymer hardly moves extremely or falls off.

How stably a superabsorbent polymer is embeddedly supported can further be evaluated as follows. Fifty grams of physiological saline (0.9 wt% NaCl aqueous solution) is evenly applied onto the specimen that has been subjected to the above-described shaking test to observe the swell. When the variation in thickness of the swollen specimen is such that the maximum is not larger than double the minimum, it is safe to say that the polymer hardly moves extremely or falls off.

Note that the specimen to be used in the above-described methods of evaluation should be cut out of an area wherein the superabsorbent polymer is sprinkled uniformly in the planar direction of the web.

In case where the web has insufficient polymer supporting properties, it is possible to add to the web an appropriate amount of a hot-melt adhesive, a binder of various kinds (e.g., acrylic emulsion pressure-sensitive adhesive), sugar derivatives (e.g., carboxymethyl cellulose and ethyl cellulose) or a thermoplastic resin (e.g., polyethylene, polypropylene or polyethylene terephthalate). An embossed sheet or a flock-finished sheet may be used in combination.

The total weight of the webs 2 and the polymer layer(s) 3 of the absorbent member 1 is preferably 120 to 400 $g/m^2$, more preferably 150 to 300 $g/m^2$, for application to disposable diapers; preferably 35 to 200 $g/m^2$, more preferably 50 to 150 $g/m^2$, for application to sanitary napkins; or preferably 35 to 500 $g/m^2$, more preferably 50 to 400 $g/m^2$, for application to incontinence pads.

The distribution of the superabsorbent polymer in the thickness direction of the absorbent member 1 is as described. The distribution in the planar direction will be described below. A typical distribution is a uniform distribution. In an alternative distribution, the superabsorbent polymer is localized in the front portion of the absorbent member as illustrated in Figs. 2(a) and 2(b). In Fig. 2(a) the amount of the sprinkled polymer is even in the width direction but increases toward the front end of the absorbent member. In Fig. 2(b) the polymer is sprinkled in the shape of letter U open to the rear end of the absorbent member. An absorbent member having the superabsorbent polymer localized in the front portion thereof is highly preventive against leakage from the front side of a wearer's body. Such an absorbent member is particularly suited for use in pull-on type disposable diapers.

In still another alternative distribution, the superabsorbent polymer is localized in the rear portion of the absorbent member as illustrated in Fig. 3, in which the polymer is sprinkled in the shape of letter U open to the front end of the absorbent member. An absorbent member having the superabsorbent polymer localized in the rear portion thereof is highly preventive against leakage from the rear side of the wearer's body. Such an absorbent member is particularly suited for use in disposable diapers for younger babies, patients or adults.

In yet another alternative distribution, the superabsorbent polymer is localized in the central portion of the absorbent member as illustrated in Fig. 4. Not disposed in the peripheral portion, the superabsorbent polymer is effectively prevented from falling off the absorbent member.

The distribution of the superabsorbent polymer may be varied in the width direction of the absorbent member in place of, or in addition to, the planar distribution shown in Figs. 2 through 4. For example, the weight of the superabsorbent polymer layer sprinkled along the longitudinal centerline may be increased over that in the longitudinal both side portions. An absorbent member having such a distribution of the superabsorbent polymer effectively prevents leakage from the leg openings of the absorbent article and is particularly suited for use in disposable diapers. To the contrary, the weight of the superabsorbent polymer layer sprinkled along the longitudinal both sides of the webs may be increased over that along the longitudinal centerline to enhance the spot absorptivity of the absorbent member. Such an absorbent member is particularly suited for use in incontinence pads or sanitary napkins. The above-described distribution of the superabsorbent polymer in the width direction of the absorbent member is preferably made in at least the crotch portion of the absorbent member 1.

The long fibers making up the webs 2 are unidirectionally oriented in the planar direction of the absorbent member 1. Owing to the unidirectional fiber orientation, liquid absorbed by the absorbent member 1 diffuses preferentially in the orientation direction of the long fibers, i.e., in the planar direction of the absorbent member 1. Liquid diffusion in the direction perpendicular to the orientation direction of the long fibers is suppressed. When the long fibers are oriented in the length direction of the absorbent article, side leakage is effectively prevented.

The orientation of the long fibers is defined by a vector which is formed by connecting the one end of the fiber and the other end of the fiber. Where the vector is orientated in the planner direction of the absorbent member, the long fibers are said to be oriented in the planner direction of the absorbent member. Even if part of the long fiber, which is located between the both ends, is oriented in the thickness direction of the absorbent member, the long fiber is said to be oriented in the planner direction of the absorbent member as far as the above-defined vector is oriented in the planner direction of the absorbent member. The degree of orientation of the long fibers is preferably 1.2 or higher, more preferably 1.4 or higher, as measured with a microwave molecular orientation analyzer MOA-2001A manufactured by Kanzaki Co., Ltd. Measurement is made on three points of a specimen measuring 50 mm in width and 100 mm in length to obtain an average. Where a sample is smaller than that size, two or more samples are aligned without overlap.

Among measures for securing side leakage prevention is use of a web of straight-linear long fibers; for such long fibers are superior to crimped long fibers in fluid distribution ability in the direction of their orientation. Making use of this advantage, it is preferred that at least one of the webs 2 have a part of its crimped long fibers straightened. For example, when the absorbent member 1 has two webs 2, the crimped long fibers located in part of the topsheet side web 2, e.g., in the longitudinal middle portion of the topsheet side web 2 can be straightened so that a body fluid may be led preferentially in the longitudinal direction of the absorbent member 1.

The same effect can be produced by using a web of hydrophilic long fibers with no crimp in addition to the webs 2 of crimped long fibers. For example, a web of long fibers with no crimp may be superposed on the upper side of the laminate having two webs 2 of crimped long fibers. In this case, a polymer layer may be interposed between the web with no crimp and the upper web with crimp.

Where the long fibers are oriented in the length direction of an absorbent article, it is advisable that there be no linear joint across the orientation direction of the long fibers in the absorbent member. A linear joint across the long fiber orientation direction would block smooth fluid distribution in that direction, which can cause side leaks.

Where the long fibers are oriented in the width direction of an absorbent article, spot absorptivity is obtained with longitudinal fluid distribution suppressed. In this case, it is preferred that the absorbent member have a linear joint across the orientation direction of the long fibers to prevent side leakage. The term "linear" in "linear joint" does not always mean a continuous solid line as long as it functions substantially as a continuous line that can suppress penetration of a liquid. For instance, when sealing lines formed spacedly but with overlaps when seen from a certain direction are capable of hindering smooth migration of a liquid in that direction, the sealing lines can be said to be "a linear joint". The linear joint may be curved or piecewise straight as well as straight The linear joint width is preferably 0.2 to 15 mm.

A linear joint may be formed only in the webs 2. Where the laminate containing the webs 2 is wrapped in tissue paper, etc. to complete the absorbent member 1, a linear joint may be formed through the whole thickness of the absorbent member 1. A linear joint may be such that the topsheet is joined to the absorbent member. In any configuration, a linear joint is preferably formed in the longitudinally middle portion of the absorbent article. A linear joint may be formed outboard of both lateral side edges of the absorbent member. If a body fluid diffuses in the web through capillarity, it will run into the linear joint and is blocked from further diffusion thereby prevented from causing side leakage.

According to the above since the superabsorbent polymer is retained in the spaces formed by the crimped long fibers, the polymer hardly moves extremely or falls off, and the absorbent member 1 is hardly destroyed by active movement of a wearer. For the purpose of further enhancing these effects and reducing crispness generated by the superabsorbent polymer rubbing against itself, the superposed webs 2 are bonded to each other. In order not to interfere with preferential liquid distribution in the orientation direction of the long fibers, the superposed webs 2 are bonded in points. The expression "bonded in points" indicates that the individual joints do not have appreciable anisotropy and that such joints are uniformly dispersed in every planar direction of the webs 2. Typically, superposed webs 2 are joined via a large number of small dots. Bonding in points can be achieved by, for example, spraying a hot-melt adhesive by slot spraying, curtain spraying, melt blown spraying or helical spraying. The thus applied adhesive can bond the neighboring webs 2 not only to each other but also to part of the superabsorbent polymer.

The absorbent member 1 may be composed solely of a combined layers of the webs 2 and the polymer layer 3. Otherwise, the combined layers may be wrapped in a sheet material, or a sheet material may be superposed on the upper side and/or the lower side of the combined layers. The combined layers covered with the sheet material may further be wrapped in another sheet material. Sheet materials used in these structures include paper made of fluff pulp, tissue paper, a dry processed pulp sheet, and nonwoven fabrics (e.g., air-through nonwoven fabric and airlaid nonwoven fabric). For use in disposable diapers, the absorbent member 1 is of thin type preferably with a thickness of 1 to 4 mm, more preferably 1.5 to 3 mm, whichever structure it may have. For use in sanitary napkins, the thickness of the absorbent member is preferably 0.5 to 3 mm, more preferably 1 to 2 mm. For application to incontinence pads, the thickness is preferably 0.5 to 4 mm, more preferably 1 to 3 mm.

The thickness of the absorbent member 1 is measured with a load of 2.5 g/cm$^2$ applied thereto by placing a 5 cm square acrylic resin plate and a weight. In the present embodiment, the thickness is measured with a laser displacement sensor LK080 class 2 available from Keyence Corp. Measurement is made at five points to obtain an average. If a piece of data fluctuates 20% or more, the piece is replaced with an additional one. Prior to the measurement, a load of 250 $g/cm^2$ is applied for 12 hours to the sample to straighten wrinkles.

A preferred process of producing the absorbent member 1 of the first embodiment will be described. First of all, a tow of long fibers having the above-specified crimp percentage is prepared. The tow is transported while being stretched under tension in the machine direction. In this state, the tow is opened into a web. The step of opening the tow is achieved by using, e.g., an air opening apparatus utilizing compressed air. Having crimp, the long fibers are easily stretched out under tension in the machine direction. A superabsorbent polymer is sprinkled on the stretched web. Before sprinkling, the running speed of the web is slowed down, and the web is transferred onto a vacuum conveyor. The web on the vacuum conveyor is released from the tension. On release from tension, the long fibers return to their crimped state and, at the same time, the web becomes bulkier than under tension and gains improved ability to retain the superabsorbent polymer. In this state, the superabsorbent polymer is sprinkled. The crimped longer fibers have interfiber spaces in which the superabsorbent polymer can be embedded and retained. In this fashion, a desired amount of the superabsorbent polymer can be embeddedly supported in the web. In contrast, it is difficult to embed and retain the superabsorbent polymer in the web in the stretched state under tension; for the stretched web is incapable of providing ample space between fibers enough to hold the polymer. It is effective in helping the polymer be embeddedly retained to suck the web from the reverse side simultaneously with the polymer sprinkling. It is possible to vary the polymer distribution in the web thickness direction by appropriately adjusting the degree of suction.

Alternatively the superabsorbent polymer may be sprinkled on the web 2 of the opened tow in a state stretched to a predetermined length. In this process the long fibers do not need to be straightened completely. It is sufficient to stretch the web 2 to such an extent as to allow the superabsorbent polymer to be embeddedly retained in the web 2 in a stable manner.

Prior to the step of sprinkling the superabsorbent polymer, an adhesive such as a hot-melt adhesive is applied to the web 2 having the long fibers stretched. The step of applying the adhesive is carried out either by a contact type application system such as a roll coater system or a screen printing or a non-contact type application system such as spray coating. Spray coating, a non-contact application system, is preferred for easy changeover of application patterns and adjustability of the amount of the adhesive to be applied. In particular, a spray coating system fit for bonding in points is preferred. Spray coating systems include slot spraying, curtain spraying, melt blown spraying, and helical spraying. The amount of the adhesive to be applied is preferably as small as not to become a hindrance to migration of liquid between the webs. From this viewpoint, the amount of the adhesive to be applied preferably ranges from 3 to 30 $g/m^2$, more preferably 5 to 15 $g/m^2$.

After the adhesive is applied, the superabsorbent polymer is sprinkled on the web 2. After completion of the polymer sprinkling, another, separately prepared web 2 is superposed thereon. The long fibers are then released from the stretched state, whereupon the long fibers contract. It follows that the superabsorbent polymer is successfully retained in the spaces formed by the contracted long fibers and thus embeddedly supported in the webs 2. The two webs 2 are thus bonded together in points.

In a modified process, the web 2 on which the superabsorbent polymer has been sprinkled is released from the stretched state to prepare a plurality of intermediate webs in which the long fibers have been let to contract, which are finally bonded to one on another to obtain the absorbent member 1. In this case, the uppermost layer is the polymer layer 3. Extreme movement or fall-off of the polymer, which is very likely to occur, can be avoided by wrapping the whole of the resulting absorbent member 1 in tissue paper, nonwoven fabric, etc.

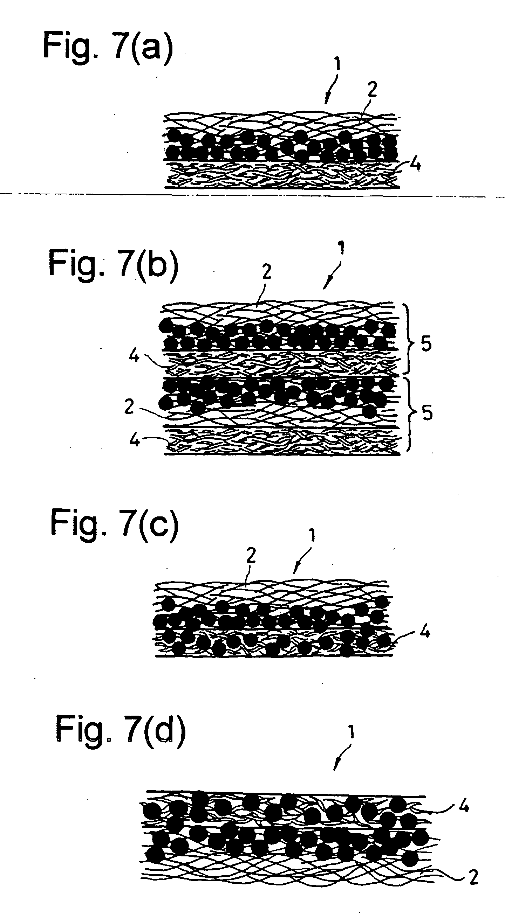

When the absorbent member 1 has the structure of Fig. 7(c) or 7(d) described infra, i.e., a laminate structure composed of a web of long fibers in which a superabsorbent polymer is embeddedly supported and an airlaid fiber layer containing pulp and a superabsorbent polymer, the following process of production can be adopted.

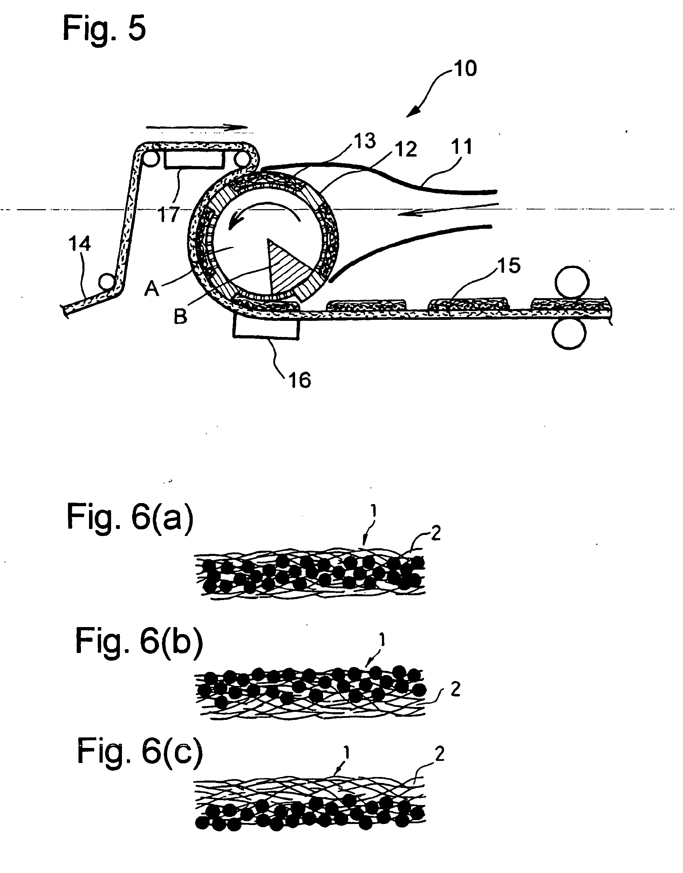

The apparatus illustrated in Fig. 5 is used to carry out the process. The apparatus 10 shown in Fig. 5 has a rotating drum 12 with a hood 11. The drum 12 has many recesses 13 engraved on its periphery at a regular interval along the direction of rotation. The bottom of each recess 13 is formed of an air permeable material. The inside of the drum 12 is connected to a suction source (not shown) so that air is sucked from the outside to the inside of the drum 12 through the air permeable material. The inside of the drum 12 is partitioned into regions A and B. When the recess 13 is positioned on the periphery of the region A, the recess 13 is sucked through the air permeable bottom. When the recess 13 is positioned on the periphery of the region B, the recess 13 is not sucked.

Pulp and a superabsorbent polymer fed from a feeder (not shown) are carried by air currents and successively deposited in the recesses 13. Separately, an unrolled web of long fibers is transported in a longitudinally stretched state under tension and opened evenly into a web 14 by means of an opening apparatus (not shown). The web 14 is conveyed to and wrapped around the drum 12. When an airlaid fiber layer 15 of pulp and a superabsorbent polymer accumulated in the recess 13 comes to the region B of the rotating drum 12, it is released from the suction and transferred onto the web 14. A suction box 16 is installed beneath the web 14 right under the position where the airlaid fiber layer 15 is transferred to the web 14 to ensure the transfer. In this way the airlaid fiber layer 15 is superposed on the web 14.

The superabsorbent polymer is fed to the rotating drum 12 in large excess with respect to the pulp. With this difference in amount between the superabsorbent polymer and the pulp, a nearly equivalent weight mixture of the pulp and the superabsorbent polymer is formed and accumulated in the recess 13 to form the airlaid fiber layer 15, and the excess of the superabsorbent polymer is then deposited on the surface of the airlaid fiber layer 15. By transferring the airlaid fiber layer 15 to the web 14, the superabsorbent polymer on the surface of the airlaid fiber layer 15 is embeddedly supported by the web 14. In order for the superabsorbent polymer to be embeddedly supported more effectively, the web 14 is sucked onto the conveyer by a suction box 17 so as to reduce the tension applied to the web 14 before being wrapped around the drum 12. It follows that the web is released from the stretched state whereupon the long fibers return to their original crimped state. In this condition, the excess of the superabsorbent polymer on the airlaid fiber layer 15 is transferred to the web 14 and embedded in the spaces between crimped long fibers. Suction by the suction box 16 placed downstream the drum 12 further ensures the superabsorbent polymer to be embedded firmly.

The above-described process provides with ease the absorbent member composed of the long fiber web 14 in which the superabsorbent polymer is embeddedly supported and the airlaid fiber layer 15 containing pulp and the superabsorbent polymer. The process is efficient in that the superabsorbent polymer can be incorporated into both the web 14 and the airlaid fiber layer 15 by feeding the superabsorbent polymer through a single feed line.

The process offers another advantage that the particle size of the excess superabsorbent polymer deposited on the surface of the airlaid fiber layer 15 is controllable. In general, the superabsorbent polymer has a particle size distribution. Transportability of the superabsorbent polymer as supported in an air flow varies depending on the particle size. Therefore, polymer particles of small sizes are liable to be blended with pulp, while those of large sizes are liable to be deposited on the airlaid fiber layer 15. In other words, relatively finer polymer particles are present in the airlaid fiber layer 15, and relatively larger polymer particles are present in the web 14. The absorbent member 1 composed of the web 14 and the airlaid fiber layer 15 with such a polymer particle size gradient, exemplified by the configuration shown in Fig. 7(c), exhibits an increased rate of liquid penetration.

Other embodiments of the first aspect of the present invention will then be described with reference to Figs. 6 through 10. The description provided above with reference to the first embodiment shown in Fig. 1 applies appropriately to those particulars of the other embodiments that are not described here. Elements in Figs. 6 to 10 identified with the same numerals as in Figs. 1 to 4 may be identical and will not be redundantly described.

The absorbent member 1 illustrated in Fig. 6(a) is comprised of a single web 2 in which a superabsorbent polymer is uniformly embeddedly supported. The absorbent members 1 illustrated in Figs. 6(b) and 6(c) are also comprised of a single web 2 but different from the embodiment of Fig. 6(a) in that the superabsorbent polymer is localized in the thickness direction. The superabsorbent polymer is localized in the skin-facing side of the web 2 in Fig. 6(b) or the garment-facing side of the web 2 in Fig. 6(c). The amount of the superabsorbent polymer may vary in the thickness direction either continuously or stepwise. The absorbent member 1 of Fig. 6(b) exhibits high spot absorptivity and is fit for use in pads for light incontinence or sanitary napkins. The absorbent member 1 of Fig. 6(c) exhibits excellent liquid distribution and high liquid absorptivity as a whole and is therefore fit for use in disposable diapers.

The absorbent member 1 illustrated in Fig. 7(a) has an airlaid fluff pulp layer 4 and a web 2 in which a superabsorbent polymer is embeddedly supported. Because the airlaid fluff pulp layer 4 functions as a temporary liquid reservoir, the absorbent member 1 effectively prevents leakage even when a body fluid is discharged at a high speed as in urination. From that viewpoint, it is preferred for the superabsorbent polymer embeddedly supported in the web 2 to be localized in the garment facing side of the web 2. It is also possible that the superabsorbent polymer is sprinkled in the fashion illustrated in Figs. 6(a) or 6(b). The absorbent member 1 of Fig. 7(a) is suited for use in disposable diapers.

The absorbent member 1 illustrated in Fig. 7(b) has a plurality of laminates 5 stacked one on top of another, each laminate 5 has an airlaid fluff pulp layer 4 and a web 2. The absorbent member 1 of Fig. 7(b) is more leakproof than that of Fig. 7(a).

The absorbent member 1 illustrated in Fig. 7(c) is similar to that of Fig. 7(a), except that a superabsorbent polymer is mixed into the airlaid fluff pulp layer 4. The airlaid fluff pulp layer 4 performs an enhanced function as a temporary liquid reservoir. As is apparent to those skilled in the art, the structure illustrated in Fig. 7(b) may be fabricated using a plurality of the absorbent members 1 of Fig. 7(c) or a combination of the absorbent members of Fig. 7(a) and 7(c).

The absorbent member 1 illustrated in Fig. 7(d) is a vertical inversion of the structure illustrated in Fig. 7(c). An absorbent article using the absorbent member 1 of Fig. 7(d) will retain satisfactory absorptivity when the wearer's body pressure is imposed, for example, when the wearer is in a lying or sitting posture.

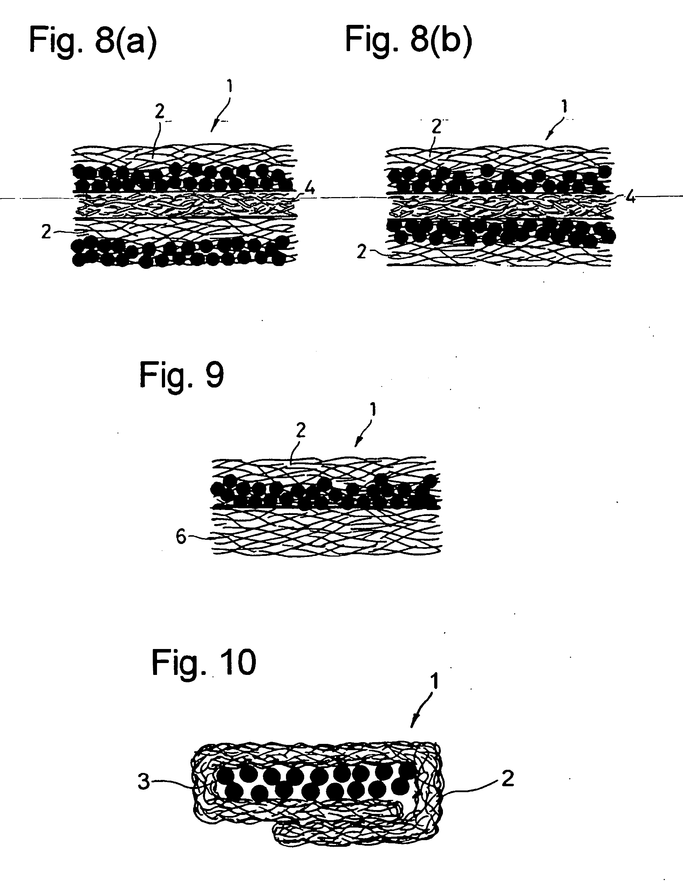

The absorbent members 1 illustrated in Figs. 8(a) and 8(b) each have an airlaid fluff pulp layer 4 and a web 2 superposed on each side of the airlaid fluff pulp layer 4. In the structure of Fig. 8(a), the superabsorbent polymer embeddedly supported in each web 2 is localized in the garment facing side. In the absorbent member of Fig. 8(b), the superabsorbent polymer embeddedly supported in the upper web 2 is localized in the garment facing side while that in the lower web 2 is localized in the skin facing side. The structure of Fig. 8(a) allows for a body fluid being temporarily reserved. Besides, since the liquid distributing layer exists on the skin facing side and in the middle of the absorbent member, the fluid that has not been caught by the upper layer side is further diffused in the lower layer side. As a result, the superabsorbent polymer enjoys high usability, making it possible to quickly absorb a large amount of a body fluid discharged at a high rate. Accordingly, the absorbent member according to the embodiment shown in Fig. 8(a) is fit for use in pull-on diapers, training pants or underwear for night time for older babies or toddlers. On the other hand, because the absorbent member illustrated in Fig. 8(b) has a mechanism that a body fluid once reserved in the airlaid fluff pulp layer 4 is fixed by the superabsorbent polymer disposed on both sides of the fluff pulp layer 4, it is difficult for this type of absorbent member to receive a body fluid discharged at a high rate. However, the interfiber spaces provided on the outermost garment facing side develop cushioning effects and allow for designing diapers with an improved hand. Furthermore, the absorbent member has reduced rewet because the body fluid is finally fixed in the middle of the thickness of the absorbent member. Therefore, the absorbent member 1 according to the embodiment of Fig. 8(b) is suitable for use in diapers for younger babies.

The absorbent member 1 shown in Fig. 9 has a web 2, in which a superabsorbent polymer is embeddedly supported, and a web 6 made up of hydrophilic long fibers and containing no superabsorbent polymer. The web 6 is disposed underneath the web 2. Where the long fibers of the web 6 are crimped fibers, particularly those having the above recited specific crimp percentage, the web 6 functions as a cushioning layer against pressing in the thickness direction and therefore provides improved wearing comfort.

The absorbent member 1 shown in Fig. 10 has a long fiber web 2 folded in three along the longitudinal direction and a superabsorbent polymer 3 held in the fold. The absorbent member 1 of Fig. 10 has an advantage that the superabsorbent polymer is supported more securely. In addition, the absorbent member 1 provides an absorbent article with a soft and fluffy feel with little crispness of the superabsorbent polymer felt when touched from either of the topsheet and the backsheet sides. The overlap of the web provides an increased thickness, an improved fluffy feel, and an improved fit to a wearer's body. Also performing the function as a temporary liquid reservoir, the overlap is effective for quick absorption and leak prevention.

The absorbent member 1 of Fig. 10 may have retained in its fold an airlaid absorbent core containing hydrophilic short fibers, the superabsorbent polymer, and a thermoplastic synthetic pulp or binder (e.g., polyvinyl acetate or an acrylic emulsion). Being stiff, the airlaid absorbent core gives stiffness to an absorbent article and also exhibits a high rate of absorption under pressure. If the airlaid absorbent core is used alone as an absorbent member, however, the stiffness can cause a wearer a discomfort or give rise to a skin trouble due to friction and sometimes lacks fluffiness. Then, a part or the whole of the airlaid absorbent core is covered with the web 2 to reduce the demerits while retaining the merits of the airlaid absorbent core.

A sanitary napkin and an incontinence pad are generally smaller in size as compared with a disposable diaper. Hence, in some cases, a region having an increased absorption capacity, hereinafter "high absorption region", is provided in part of the product to impart spot absorptivity. When any of the foregoing embodiments is employed as a high absorption region, the high absorption region preferably has a convex shape. In this application, the web 2 preferably weighs 100 to 1000 g/m$^2$, more preferably 200 to 500 g/m$^2$, and the superabsorbent polymer preferably weighs 50 to 1000 g/m$^2$, more preferably 100 to 500 g/m$^2$.

In each of the embodiments described above, the density of the web is preferably 0.005 to 0.20 g/m$^3$, more preferably 0.01 to 0.10 g/m$^2$. With the density being in that range, it is possible to control the rate of liquid penetration within a range appropriate for the absorption rate of the superabsorbent polymer; the softness as an absorbent member is retained; and the web has a moderate interfiber distance for securing improved property of supporting fine superabsorbent polymer particles.

It is preferred for the superabsorbent polymer used in the foregoing embodiments to have a water (physiological saline) absorption of 30 g/g or more, more preferably 30 to 50 g/g, measured by a centrifugal dewatering method, taking into consideration the amount of the superabsorbent polymer to be used and prevention of reduction in gel feel after liquid absorption. The absorption measurement by the centrifugal dewatering method is carried out as follows. One gram of a superabsorbent polymer is swollen with 150 ml of physiological saline for 30 minutes and put in a 250-mesh nylon bag. The bag containing the swollen polymer is dewatered in a centrifuge at 143G (800 rpm) for 10 minutes and weighed. The water absorption (g/g) is calculated according to equation:

Water absorption by centrifugal dewatering method =
(total weight after dewatering − weight of dry nylon bag − weight of superabsorbent polymer before absorption − weight of liquid remaining in wet nylon bag)/weight of superabsorbent polymer before absorption

It is preferred for the superabsorbent polymer used in the foregoing embodiments to have a liquid transit time of 20 seconds or less, more preferably 2 to 15 seconds, even more preferably 4 to 10 seconds, measured by the following method. With the liquid transit time falling within that range, the phenomenon called gel blocking and the resultant reduction in absorptivity are prevented from occurring. If the absorption rate of the polymer layer fails to keep up with the discharged liquid, the excess liquid will pass through the polymer layer and can leak. Such leakage is prevented by controlling the liquid transit time within the above range. The liquid transit time is measured as follows. A cylinder having a cross-sectional area of 4.91 $cm^2$ (inner diameter: 25 mm) with its bottom closable with a cock (inner diameter: 4 mm) is prepared. In the cylinder with its bottom closed is put 0.5 g of a superabsorbent polymer, and the cylinder was filled with physiological saline. After the polymer is swollen to saturation and sinks to the bottom, the cock is opened to have the physiological saline pass through the swollen polymer. The time required for 50 ml of the saline to pass through is taken as the liquid transit time. The liquid transit time is a measure of the gel strength of the superabsorbent polymer. The shorter the liquid transit time, the higher the gel strength.

The superabsorbent polymer that can be used in the foregoing embodiments is not particularly limited as long as the above-mentioned various characteristics are satisfied. Examples of suitable superabsorbent polymers include sodium polyacrylate, acrylic acid-vinyl alcohol copolymers, crosslinked sodium polyacrylate, starch-acrylic acid graft copolymers, isobutylene-maleic anhydride copolymers and saponification products thereof, potassium polyacrylate, and cesium polyacrylate. In order for the superabsorbent polymer to satisfy the characteristics, a crosslinking density gradient is provided on the surface of the polymer particles. Specifically, the method disclosed in JP-A-7-184956 can be used.

Compared with conventional absorbent members containing fluff pulp as a main fibrous material, the absorbent member of the present invention, which uses a web of long fibers, forms a network structure having ample spaces between the fibers. As a result, the absorbent member allows for smooth liquid passage. When the superabsorbent polymer has a low rate of absorption, it can happen that a body fluid passes through the absorbent member before being absorbed by the superabsorbent polymer and is not sufficiently absorbed by the absorbent member. To avoid this, it is desirable for the superabsorbent polymer used in the present invention to have a sufficiently high rate of absorption so that a body fluid may be surely retained in the absorbent member. The absorption rate of a superabsorbent polymer is represented by the value (ml/0.3 g·30 sec) obtained by the demand wettability (DW) method using a DW tester generally known for carrying out the DW method, in which, with the liquid levels of physiological saline being equal, 0.3 g of a superabsorbent polymer is scattered on a mount (diameter: 70 mm; No. 1 glass filter having placed thereon No. 2 filter paper), and the water absorption after 30 seconds is gauged by reading the scale on the buret indicating a drop of the liquid level of physiological saline (the water absorption at the time of scattering the polymer is taken zero). The absorption rate of a superabsorbent polymer can be adjusted by the particle shape and size, bulk density, crosslinking degree, etc.

In the embodiments in which the absorbent member contains no or, if any, not more than 30% by weight of, pulp, it is preferred to use a superabsorbent polymer having an absorption rate of 2 to 10 ml/0.3 g·30 sec, more preferably 4 to 8 ml/0.3 g·30 sec, measured by the DW method. In the production of conventional absorbent members made mainly of fluff pulp, the use of a superabsorbent polymer having such a high absorption rate has been avoided for fear of inducing gel blocking which leads to leakage. In the above-identified embodiments, since the web exhibits high liquid-take-up properties and allows the taken up liquid to pass through at a high speed owing to its sparse structure, the superabsorbent polymer having such a high rate of absorption hardly causes gel blocking and, on the contrary, effectively prevents leakage. The weight of the absorbent member as referred to herein is inclusive of the weight of a covering or wrapping sheet.

As described above, a superabsorbent polymer having a short liquid transit time or a high absorption rate may be used alone or may be used as a mixture with or in combination with another superabsorbent polymer whose liquid transit time or absorption rate falls in the above-specified preferred range. For example, a superabsorbent polymer S1 having a relatively short liquid transit time and a superabsorbent polymer S2 having a relatively long liquid transit time can be used as a mixture. Comparing the superabsorbent polymers S1 and S2, the superabsorbent polymer S2 has a higher absorption capacity and absorption rate but is less resistant to gel blocking. In the system containing both the superabsorbent polymers S1 and S2, the superabsorbent polymer S1, which is harder and hardly induces gel blocking, enters between the particles of the superabsorbent polymer S2 having high absorbent performance. As a result, the absorbent member can be made more effective use of. As another example, a superabsorbent polymer S3 having a relatively high absorption rate and a superabsorbent polymer S4 having a relatively low absorption rate may be used in combination. In this example, the superabsorbent polymer S3 and the superabsorbent polymer S4 are disposed on the backsheet side and the topsheet side, respectively, thereby increasing the liquid take-up speed of the absorbent member and enhancing liquid fixing performance. In still another example, the same effect is obtained by disposing the superabsorbent polymer S1 having a short liquid transit time and the superabsorbent polymer S3 having a high absorption rate on the topsheet side and the backsheet side, respectively.

By using the superabsorbent polymers having the above described specific absorption characteristics, the absorbent member according to the present invention has reduced rewet notwithstanding the thinness and softness. The amount of rewet is preferably 1 g or less, more preferably 0.5 g or less, even more preferably 0.25 g or less, as measured as follows. FD & C Red No. 1 is added to physiological saline in a concentration of 50 ppm (0.5 g per 10 liters of physiological saline). The colored saline weighing 160 g is poured on the widthwise middle portion 150 mm below the frontal waist edge of a medium size disposable diaper for infants by use of a funnel. Ten minutes after completion of the pouring, a stack of ten sheets of filter paper No. 4A available from Advantech Toyo Kaisha, Ltd. is placed on the wet portion, and a load of 3.43 kPa is applied thereon for 2 minutes to have the filter paper absorb the saline. The filter paper is weighed, and the weight gain is taken as the amount of rewet Measurement is made three times to obtain an average. When a diaper of other size is tested, the following alterations are made. In the case of disposable diapers for infants, the load applied to the filter paper being fixed at 3.43 kPa, the amount of the saline to be poured is changed according to size (120 g for newborn and S size diapers, 160 g for other sizes). In the case of absorbent articles for adults inclusive of sanitary napkins, the load applied to the filter paper is fixed at 5.15 kPa. In testing sanitary napkins, 10 g of horse blood is used in place of the colored physiological saline.

The web may contain other organic or inorganic particles than the superabsorbent polymer, including activated carbon, silica, alumina, titanium oxide, and various clay minerals (e.g., zeolite, sepiolite, bentonite, and cancrinite) that can serve as deodorants or antimicrobials. The inorganic particles may have part of the metal sites displaced. Various organic or inorganic buffers can also be used, including acetic acid, phosphoric acid, citric acid, succinic acid, adipic acid, malic acid, lactic acid, and salts of these acids, either individually or as a mixture thereof. Various amino acids are also used. The function of these ingredients is to suppress the smell of discharged body waste and the smell of the constituting materials per se. The organic or inorganic buffers also have a function of neutralizing body waste, for example ammonia generated by decomposition of urine to maintain the diaper neutral to weakly acidic, which minimizes the skin irritation even if rewet occurs. Furthermore, the organic or inorganic buffers, being capable of neutralizing an alkali (e.g., ammonia), is expected to protect such long fibers as have an intermolecular ester bond (e.g., cellulose acetate fiber) against damage due to ester bond cleavage by an alkali.

The web may contain hydrophilic fine powder or stable to improve the liquid retention, rate of absorption, and a dry feel. The hydrophilic fine powder or staple includes fibrillated or non-fibrillated cellulose powder, carboxymethyl cellulose and metal salts thereof, carboxyethyl cellulose and metal salts thereof, hydroxyethyl cellulose and derivatives thereof, silk powder, nylon powder, and rayon, cotton or wool staple fibers. Preferred of them is cellulose powder for being the most effective of the others. The hydrophilic fine powder or staple may be dispersed either before the superabsorbent polymer is sprinkled or as a mixture with the superabsorbent polymer.

It is preferred that the long fibers making up the web be bonded to one another to bring about improvements in shape retention, recovery from compression, resistance to bunching up, and transportability. Bonding of long fibers can be achieved by using, for example, a water soluble adhesive such as polyvinyl acetate or an acrylic emulsion.

Cellulose acetate long fibers, for example, can be bonded to one another by spraying an agent capable of dissolving or plasticizing cellulose acetate, such as triacetin, onto the web after sprinkling the superabsorbent polymer.

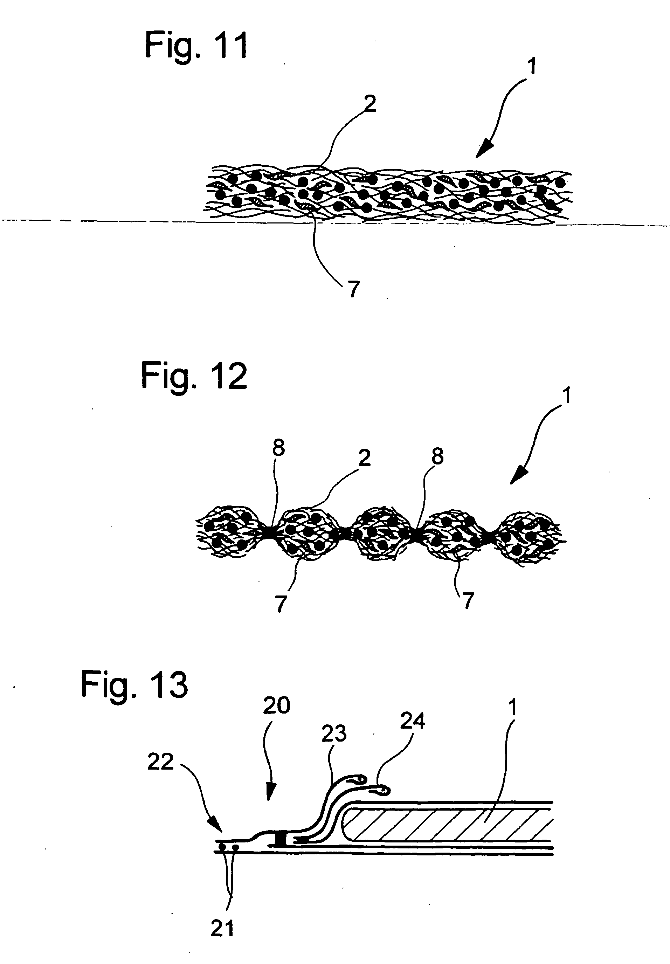

Another method for bonding long fibers is to disperse a synthetic pulp of a thermoplastic resin in the web, followed by heating to melt the synthetic pulp as illustrated in Fig. 11. The synthetic pulp 7 can be dispersed before, after or simultaneously with the sprinkling of the superabsorbent polymer. During the dispersing, the web is preferably sucked from the opposite side so that the synthetic pulp and the superabsorbent polymer may be sufficiently distributed throughout the web. When in using thermoplastic resin long fibers, it is preferred to use a synthetic pulp of a thermoplastic resin having a lower melting point than the thermoplastic resin long fibers.

It is a preferred embodiment that the absorbent member of Fig. 11 is embossed to provide the absorbent member 1 shown in Fig. 12. The absorbent member 1 of Fig. 12 has a large number of embossed, densified parts 8. That is, the web has high fiber density parts and low fiber density parts to create a capillary force difference therebetween. As a result, the absorbent member 1 of Fig. 12 has higher liquid drawing properties than that of Fig. 11.

Another method for improving the shape retention of the web is to cover or wrap the web with one or more sheet materials. The sheet material is put on the skin facing side and/or the garment facing side and/or both longitudinal side faces of the web and bonded to the web with an adhesive or by fusion. The resulting absorbent member has increased stiffness due to the joint with the sheet material as well as the stiffness of the sheet material per se. As a result, the absorbent member is easier to handle and thus easier to transport by itself Besides, the absorbent member is easy to cut or punch into a piece of any desired shape in conformity to the design of an absorbent article.

Bonding the sheet material to the web is preferably done with an adhesive, the adhesive is preferably applied in a fashion that does not impair the water infiltrability, softness, and breathability of the web. With this regard, it is advantageous to apply the adhesive in as fine a fiber as possible discretely (e.g., in a helical pattern, a linear pattern, or a continuous Ω shaped pattern) so that the long fibers may be bonded at a large number of joints without damaging the characteristics of the web. To achieve this, a hot melt adhesive application system Dyna Fiber UFD (trade name) can be used. The adhesive is not particularly limited in kind, whether hydrophilic or hydrophobic. Hydrophilic adhesives are preferred. Examples of the hydrophilic adhesives include Cycloflex, a hot melt adhesive available from National Starch and Chemical Company, Delaware, US. Generally, the sheet material and the web are bonded at only their surfaces. In some cases, part of the adhesive is impregnated into the web, and the fibers contained inside the web may be bonded by the impregnated adhesive.

Superposing the sheet material on the skin facing side and/or the garment facing side of the web is also advantageous to enhance the absorption performance of the absorbent member. The sheet materials fit for this purpose include various fiber sheets and fiber webs, such as air-through nonwoven fabric, airlaid nonwoven fabric, dry processed pulp nonwoven fabric, airlaid structure of crosslinked pulp, paper containing crosslinked pulp, and composites thereof. These sheet materials may be used either singularly or as a set of two or more. The fiber constituting the sheet material preferably has a diameter of 1.7 to 12 dtex, more preferably 2.2 to 7.8 dtex, even more preferably 3.3 to 5.6 dtex. The sheet material preferably has a basis weight of 15 to 200 $g/m^2$, more preferably 20 to 150 $g/m^2$, even more preferably 25 to 120 $g/m^2$. More specifically, when it is desired to improve the rate of liquid take-up, to prevent rewet, or to accelerate liquid distribution in the sheet material, the basis weight of the sheet material is preferably 15 to 100 $g/m^2$, more preferably 20 to 80 $g/m^2$, even more preferably 25 to 50 $g/m^2$. When it is desired to improve the cushioning properties, to prevent bunching and roping, to impart recovery from compression, or to suppress water vaporization from the absorbent member, the basis weight of the sheet material is preferably 25 to 200 $g/m^2$, more preferably 30 to 150 $g/m^2$, even more preferably 40 to 120 $g/m^2$.

A still another method for improving the shape retention of the web is to unify the long fibers making up the web and a network sheet by entanglement. The web is superposed on one or both sides of a network sheet, and the long fibers of the web are entangled with the network sheet by hydroentanglement or needle punching using hooked needles. Thereafter, the superabsorbent polymer is sprinkled and embeddedly supported in the web. The network sheet includes a synthetic resin lattice net and a perforated film with a large number of perforations. The lattice net preferably has a wire diameter of 0.1 to 3 mm and a wire-to-wire distance of 2 to 30 mm. The perforated film preferably has a hole diameter of 4 to 40 mm and a hole-to-hole distance of 1 to 10 mm.

The absorbent article according to the first aspect having any one of the above-described absorbent member embodiments may have two or more pairs of standing gathers (cuffs). For example, the absorbent article illustrated in Fig. 13 has a pair of leg flaps 20 extending outward from the side of the absorbent member 1 and elastic strands 21 disposed in each of the leg flaps in their stretched state to form a leg gather 22. The absorbent article of Fig. 13 also has a pair of first standing gathers 23 and a pair of second standing gathers 24, each having its base fixed between the leg gather 22 and the side edge of the absorbent member 1. The first standing gathers 23 are closer to the leg gathers, and the second ones to the absorbent member.

The three gathers located in each of the leg flaps 20 are preferably designed such that the outermost one may have a higher contractibility than the rest of three. That is, taking the contractive force of the leg gather 22, the first standing gather 23, and the second standing gather 24 as L1, L2, and L3, respectively, a preferred relationship is L1>L2 and L1>L3. It is more preferred that the contractive force gradually decreases from the outermost to the innermost, i.e., L1>L2>L3 for the following reasons.

Absorbent articles have been designed based on the concept that thickness reduction without causing leakage could be accomplished by providing gathers having high contractibility so as not to leave a gap between a wearer's body and the absorbent article. However, strongly contractible gathers tend to leave marks on the skin and, when combined with a thin and soft absorbent member as provided by the present invention, tend to curl up the absorbent article, making it difficult to put on the absorbent article. Moreover, too strong contractive force of gathers create downward force to cause the worn absorbent article to droop. These inconveniences associated with conventional absorbent articles can be averted by providing a pair of leg gathers and two or more pairs of standing gathers with their contractibility satisfying the above-described relationship.

The contractive force (contractibility) of a gather is measured as follows. A specimen cut out of a gather is analyzed on a tensilon tester RTC-1150A from Orientec. The specimen set at an initial span length of 100 mm is pulled to double the length (maximum length: 200 mm) and retracted to the initial length both at a speed of 300 mm/min to plot a hysteresis curve. The stress at 50 mm back from the maximum length in the declining half of the cycle (in retracting) is read as a contractive force of the specimen. Measurement is made on five specimens per sample to obtain an average. When a specimen does not extend to 200 mm, the stress required for extending the specimen to 150 mm is taken as a contractive force of the specimen.

The contractibility of the gathers can be adjusted by, for example, changing at least one of the thickness, extensibility, and the number of the elastic strands. The leg gathers 22 are preferably provided only in the crotch portion of the absorbent article.

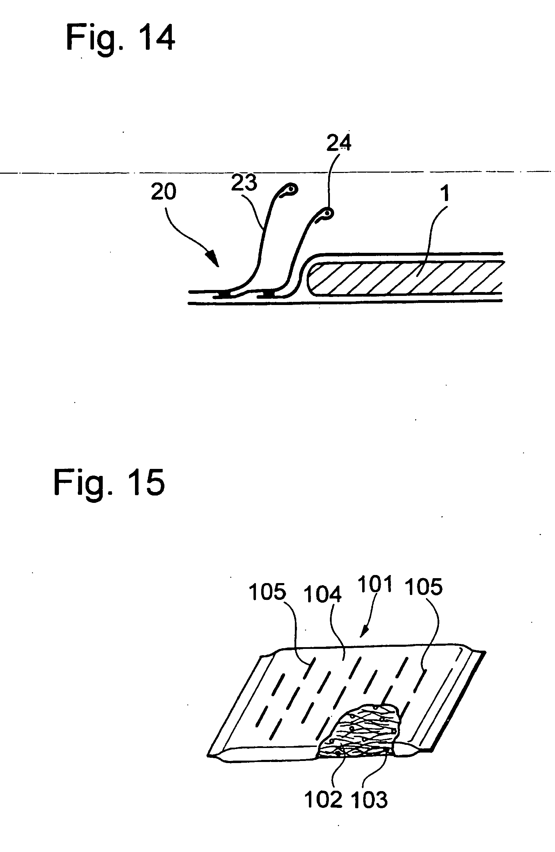

While the absorbent article illustrated in Fig. 13 has a pair of leg gathers and two pairs of standing gathers, it is possible to omit the leg gathers while retaining two or more pairs of standing gathers. For example, the absorbent article illustrated in Fig. 14 has a pair of first standing gathers 23 and a pair of second standing gathers 24. In this case, too, it is preferred that the contractibility of the standing gathers decrease inwardly for the same reasons as provided above.

The present invention will then be described with respect to the second and the third aspects. The description given above with respect to the first aspect will appropriately apply to those particulars of the second and the third aspects which are not described hereunder. Fig. 15 represents a perspective view of an embodiment of the absorbent member according to the second aspect. The absorbent member 101 illustrated in Fig. 15 is suitable for use in various kinds of absorbent articles such as disposable diapers and sanitary napkins. The absorbent member 101 has a flat, substantially rectangular shape longer than is wide. The absorbent member 101 has a hydrophilic long fiber web 102 wrapped in a liquid permeable sheet 104. The web 102 has a superabsorbent polymer 103 embeddedly supported therein. The hydrophilic long fibers are highly oriented in the longitudinal direction of the absorbent member. The absorbent member 101 exhibits stretchability in the orientation direction of the hydrophilic long fiber web 102.

The web 102 preferably has a density of 0.005 to 0.20 $g/cm^3$, more preferably 0.01 to 0.10 $g/cm^3$, to have satisfactory liquid permeability, to secure flexibility of the absorbent member 101, and to absorb a body fluid quickly.

The long fibers of the web 102 are crimped fibers. In the present embodiment, the stretchability of the long fiber web is taken advantage of to impart stretchability to the absorbent member 101. Using a long fiber web as a constituent member of the absorbent member 101 provides the absorbent member 101 with flexibility as explained below in more detail. Ground pulp is commonly used as an absorbent material of an absorbent article. Because ground pulp is a rough and stiff material, a disposable diaper using ground pulp is apt to have poor conformability to stretch and a poor fit to a wearer's body. In contrast, since a long fiber web is a flexible material, the absorbent member 101 using this has flexibility, good conformability to stretch, and a snug fit to a wearer's body.

Ground pulp generally used as an absorbent material of an absorbent article is liable to lose structural integrity as an absorbent member on liquid absorption, whereas a long fiber web is not. From this aspect, to use the long fiber web as the absorbent member 101 is advantageous.

The hydrophilic long fiber web 102 has a high degree of unidirectional orientation. Therefore, a body fluid absorbed by the absorbent member 101 is easily distributed in the orientation direction of the web 102. This property can be taken advantage of to prevent leakage. When, for example, the absorbent member 101 is disposed in an absorbent article with the orientation direction of the hydrophilic long fiber web 102 coinciding with the longitudinal direction of the absorbent article, leakage from the lateral sides of the absorbent article is prevented effectively. The definition of the orientation and the method of measuring orientation degree are as described previously.

Embeddedly supporting the superabsorbent polymer in a web made of crimped long fibers can be achieved by, for example, the following method. First of all, a web of crimped long fibers is prepared. The web is opened using, e.g., an air opening apparatus utilizing compressed air. The opened web is then stretched to a predetermined length to obtain the above-specified crimp percentage. In this step the long fibers do not need to be straightened completely. It is sufficient to stretch the web to such an extent as to allow the superabsorbent polymer to be embeddedly retained in the web in a stable manner.

While the long fibers are in the stretched state, an adhesive, such as a hot melt adhesive, is applied to the web. The pattern of application is not limited as long as the extensibility of the absorbent member 101 is not impaired. The adhesive is preferably applied in a line or dot pattern. Suitable application systems include helical spray, slot spray, control shim system (a hot melt adhesive is applied in an omega pattern), and bead coating. When a coater system is used, the adhesive can be applied in stripes. Spray coating is preferred for successfully achieving adhesion in points. The amount of the adhesive applied is as small as not to interfere with the passage of a body fluid through the web. Specifically, the amount of the adhesive is preferably 1 to 20 $g/m^2$, more preferably 2 to 10 $g/m^2$, even more preferably 3 to 7 $g/m^3$.

The adhesive may be applied in the above-described pattern all over the entire surface of the web or, part of the web surface may be left uncoated. The uncoated part will have high extensibility compared with the coated part. That is, the absorbent member may have extensibility varied in parts.

In fabricating the absorbent article using the absorbent member, it is necessary to join the absorbent member to a different member, such as a topsheet, a leakproof sheet, a sheet having an elastic member attached thereto, or an extensible sheet. Because the member that is to be joined to the absorbent member does not always have the same extensibility as the absorbent member, it is preferred that the absorbent member and the other member be joined not over the entire contact area but by partial bonding by, for example, applying a hot melt adhesive, heat fusion or ultrasonication.

After the adhesive is applied, the superabsorbent polymer is sprinkled in a layer on the web. The long fibers are then released from the stretched state, whereupon the long fibers contract to form spaces between fibers, and the superabsorbent polymer are thus embeddedly supported in the web. If desired, another, separately prepared web is superposed thereon. In this way, two webs are adhered in points.

The web 102 having the superabsorbent polymer 103 embeddedly supported therein is wrapped in the liquid permeable sheet 104. The liquid permeable sheet 104 includes a fiber sheet and a perforated film. A hydrophilic fiber sheet is preferably used as the liquid permeable sheet 104 for its satisfactory liquid permeability. The hydrophilic fiber sheet includes paper, such as tissue paper, and various nonwoven fabrics. The nonwoven fabrics include those made of hydrophilic fibers such as cotton and rayon and those made of synthetic fibers and having been rendered hydrophilic. Examples of the nonwoven fabrics are spun-bonded nonwoven fabrics, hydroentangled nonwoven fabrics, airlaid nonwoven fabrics, and air-through nonwoven fabrics. Nonwoven fabrics made of elastomeric resins, such as polyolefin resins, polyester resins or polyurethane resins, are also useful.

The weight, per unit area, of the liquid permeable sheet 104 is one of the factors influential on the liquid permeability of the sheet 104 and the flexibility and the fit of the absorbent member 101. From this standpoint, the weight of the liquid permeable sheet is preferably 5 to 250 g/m$^2$, more preferably 10 to 40 g/m$^2$.

The liquid permeable sheet 104 can be joined to the web 102 having the superabsorbent polymer 103 embeddedly supported therein by, for example, applying a hot melt adhesive to the liquid permeable sheet 104. The adhesive is applied after the superabsorbent polymer 103 is sprinkled on the web 102 and the long fibers of the web 102 are released from the stretched state. Alternatively, the liquid permeable sheet 104 may be joined via, e.g., a hot melt adhesive to the web 102 while the web 102 is in the stretched state. In the latter case, when the web 102 is released from the stretched state, the liquid permeable sheet 104 contracts concomitantly with the contraction of the web 102 in its orientation direction. The absorbent member 101 having such a structure is extensible in the orientation direction of the web 102 without subjecting the sheet 104 to any processing for extensibility development The resulting absorbent member 101 is extensible within a degree of extension of the web 102 and has an uneven surface due to the contraction of the web 102. As a result, the absorbent member 101 has an increased surface area, which contributes to improve the rate of absorption and suppress liquid spread in the longitudinal direction. Since the liquid permeable sheet 104 has no perforations, the superabsorbent polymer is hardly exposed on the surface of the absorbent member 101.

As exemplarily illustrated in Fig. 15, the liquid permeable sheet 104 may have been made extensible by a processing thereby broadening the degree of extensibility of the absorbent member 101 in the orientation direction of the web 102. More specifically, the web 102 in the absorbent member 101 is essentially stretchable owing to the crimp of the long fibers, irrespective of the orientation direction. In addition to that extensibility, if the liquid permeable sheet 104 is extensible, the absorbent member 101 will be extensible as a whole. When the absorbent member 101 in a stretched state is released from the stretched state, the web 102 contracts in the orientation direction owing to the contractibility of the crimped long fibers. Concomitantly, the liquid permeable sheet 104 also contracts. Through this mechanism, the absorbent member 101 is stretchable as a whole in the orientation direction of the web 102. The absorbent member 101 having such a structure is stretchable and has a reduced thickness. Having a flat surface, the absorbent member 101 allows a body fluid to be distributed smoothly. Since the liquid permeable sheet 104 has no perforations, it exhibits excellent absorbent performance for highly viscous fluids in a stretched state and, upon contraction, seals in the absorbed liquid. The processing for obtaining the absorbent member 101 shown in Fig. 15 is selected according to a specific design principle for an absorbent article.

The direction of extension and contraction of the absorbent member 101 does not depend on the orientation direction of the hydrophilic web 102 because the long fibers of the web 102 have crimp. That is, the web 102 is extensible in the orientation direction, the direction perpendicular to the orientation direction, and all the other directions. It is the same whether or not the long fibers are bonded to one another. The direction of extension and contraction of the absorbent member 101 depends on the direction of extensibility of the liquid permeable sheet 104 wrapping the web 102. If the liquid permeable sheet 104 is extensible in a given direction, then the absorbent member 101 is stretchable only in that direction. Where nonwoven or woven fabric is used as a liquid permeable sheet 104, which has extensibility in not only the direction of fiber orientation but in the direction perpendicular to the orientation direction, the direction of extension of the absorbent member 101 is not limited to the fiber orientation direction of the liquid permeable sheet 104. However, the absorbent member 101 has anisotropy in degree of extensibility. Where the woven or nonwoven fabric (the liquid permeable sheet 104) has not been subjected to any processing for extensibility development, the fabric has a higher maximum elongation in its fiber orientation direction than in the direction perpendicular to the fiber orientation direction.

The direction of extensibility of the absorbent member 101 is controllable by the processing that may be performed on the liquid permeable sheet 104 to make it extensible. For example, when slits are cut in the liquid permeable sheet 104 parallel with the width direction, the sheet 104 becomes extensible in the longitudinal direction so that the absorbent member 101 also becomes extensible in the longitudinal direction. When slits are cut in the sheet 1004 along the longitudinal direction, the sheet 104 becomes extensible in the direction perpendicular to the longitudinal direction, namely, the width direction so that the absorbent member 101 also becomes extensible in the width direction.

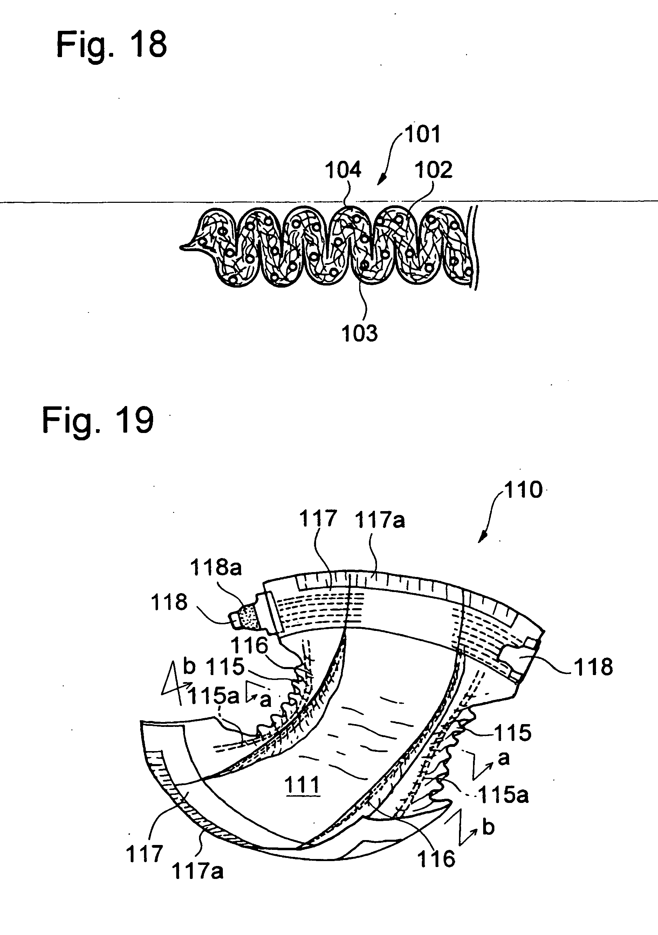

The absorbent member 101 being stretchable, an absorbent article having the absorbent member 101 provides a snug fit to a wearer's body. Appreciable improvement on fit will be displayed when the absorbent member 101 is disposed in an extensible region of an absorbent article, for example, a region where an elastic member is disposed (e.g., the leg flaps 115 illustrated in Fig. 19, described later). In that case, the absorbent member 101 is arranged with its direction showing the maximum elongation coinciding with the direction of the elastic member showing the maximum elongation.

In the present embodiment, the liquid permeable sheet 104 has been subjected to slitting (a processing of making slits in a sheet) to have many slits cut along a direction intersecting with the orientation direction of the web 102. In the embodiment illustrated in Fig. 15, the slits 105 extend in the direction perpendicular to the orientation direction of the web 102. As long as the liquid permeable sheet 104 is made extensible substantially in the orientation direction of the web 102, the extending direction of the slits 105 does not need to be exactly perpendicular to the orientation direction of the web 102.

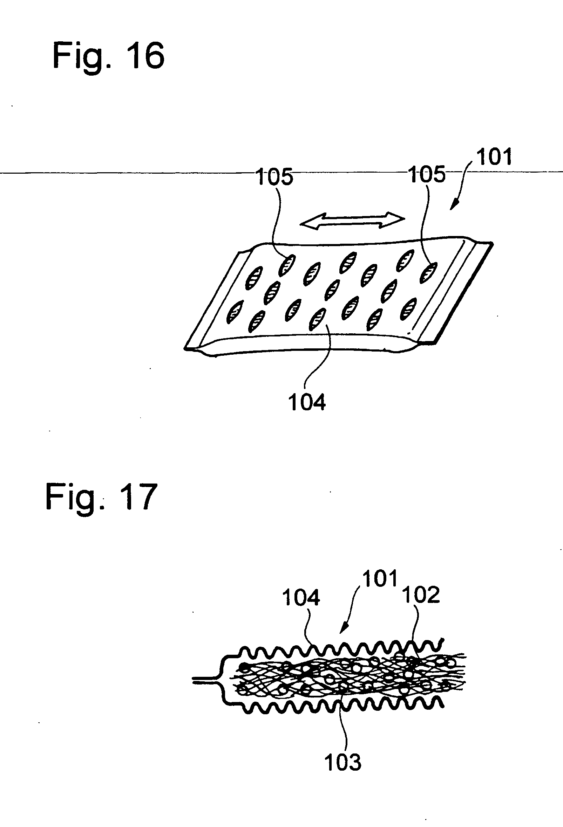

When the absorbent member 101 of Fig. 15, in which the liquid permeable sheet 104 has many slits 105 parallel with the direction perpendicular to the orientation direction of the web 102, is stretched in the orientation direction of the web 102, the slits 105 are opened to allow the liquid permeable sheet 104 to stretch, and the absorbent member 101 stretches as a whole as shown in Fig. 16.

It is preferred that the absorbent member 101 be thin and light weight to have satisfactory conformability to stretch. The thickness and the weight of the absorbent member 101 are selected appropriately depending on the use of the absorbent article in which it is used. For application to disposable diapers, for instance, the web 102 preferably has a basis weight of 5 to 200 $g/m^2$, more preferably 10 to 100 $g/m^2$, and the amount of the sprinkled superabsorbent polymer is preferably 20 to 500 $g/m^2$, more preferably 50 to 300 $g/m^2$.

For use in sanitary napkins, the web 102 preferably has a basis weight of 5 to 100 $g/m^2$, more preferably 10 to 50 $g/m^2$, and the amount of the superabsorbent polymer is preferably 10 to 200 $g/m^2$, more preferably 15 to 100 $g/m^2$. For use in incontinence pads, the web 102 preferably has a basis weight of 5 to 200 $g/m^2$, more preferably 10 to 100 $g/m^2$, and the amount of the sprinkled superabsorbent polymer is preferably 10 to 500 $g/m^2$, more preferably 15 to 350 $g/m^2$.

The total basis weight of the web 102 and the superabsorbent polymer 103 is preferably 120 to 400 $g/m^2$, more preferably 150 to 300 $g/m^2$ for use in disposable diapers; 35 to 200 $g/m^2$, more preferably 50 to 150 $g/m^2$, for use in sanitary napkins; and 35 to 500 $g/m^2$, more preferably 50 to 400 $g/m^2$, for use in incontinence pads.

The thickness of the absorbent member 101 is preferably as thin as 1 to 4 mm, more preferably 1.5 to 3 mm, for use in disposable diapers; 0.5 to 3 mm, more preferably 1 to 2 mm, in sanitary napkins; and 0.5 to 4 mm, more preferably 1 to 3 mm, in incontinence pads.

It is desirable that the absorbent member 101 be flexible. A value measured with a handle-o-meter can be used as a measure of flexibility as adopted in JIS L1096 (stiffness and softness measuring method). The "handle" of the absorbent member 101 as measured with a handle-o-meter is preferably 4 N or less, more preferably 2 N or less. The handle-o-meter is operated as follows. A specimen measuring 100 mm wide and 150 mm long is placed on the platform having a 60 mm wide slot with the length perpendicular to the slot. The force required for a 2 mm thick penetrator blade to force the center of the specimen into the slot is read. The measurement is made on three points to obtain an average. In the present invention, a handle-o-meter HOM-3 available from Daiei Kagaku Seiki Co., Ltd. was used. When in testing an absorbent member used as a side absorbent member in leg flaps as shown in Fig. 19 (described later), a sample is cut into a specimen measuring 50 mm wide and 150 mm long. Because an elastic member is usually disposed near the side absorbent member, the specimen is cut out while the leg flap is stretched to the fullest extent, and the measurement is taken on the specimen in its naturally relaxed state (as released from the stretch).

The second and the third embodiments of the second aspect will be descried by way of Figs. 17 and 18. The description provided above with reference to the first embodiment of the second aspect applies appropriately to those particulars of the second and the third embodiments that are not described here. Elements in Figs. 17 and 18 identified with the same numerals as in Figs. 15 and 16 may be identical and will not be redundantly described.

In the absorbent member 101 of the second embodiment illustrated in Fig. 17, the liquid permeable sheet 104 has been subjected to creping or pleating. The creping or pleating processing is effected to make a great number of wrinkles or pleats across the orientation direction of the web 102. Creping is a processing primarily carried on paper as a liquid permeable sheet 1004 and usually achieved by the use of a doctor blade. A preferred crepe ratio is at least 10%, more preferably at least 20%. A crepe ratio is obtained from a change in dimension when a 100 mm by 100 mm sample in an ordinary condition is dipped in water and taken out of water. A crepe ratio is calculated according to equation:

$$\text{Crepe ratio (\%)} = (\text{dimension after dipping/dimension before dipping}) \times 100$$

Pleating is a processing primarily conducted on nonwoven fabric as a liquid permeable sheet 104. A creped or pleated liquid permeable sheet embraces looseness because of the wrinkles or pleats and is therefore capable of stretch until the wrinkles or pleats are flattened.

In the third embodiment illustrated in Fig. 18, the absorbent member 101 is corrugated. That is, the web 102 and the liquid permeable sheet 104 constituting the absorbent member 101 are corrugated all together. The direction of corrugation is the orientation direction of the web 102. The corrugated absorbent member 101 is obtained by passing the web 102 wrapped in the liquid permeable sheet 104 between a pair of mating gears to shape both the web 102 and the liquid permeable sheet 104 into ripples. The thus corrugated liquid permeable sheet 104 exhibits extensibility because of its rippled shape.

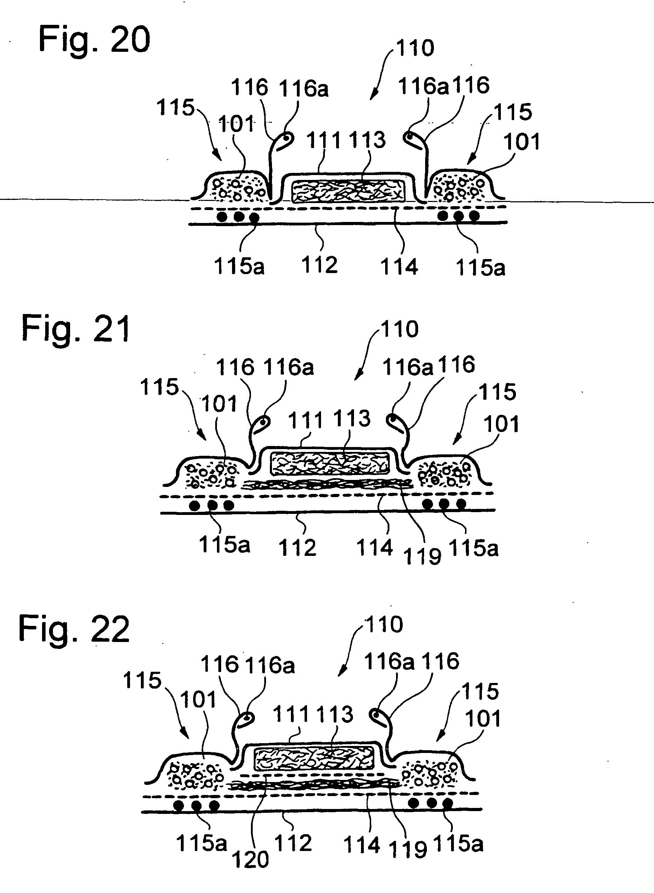

The third aspect is described with reference to Figs. 19 to 22. Fig. 19 is a perspective of a disposable diaper 110 as an embodiment of the absorbent article according to the third aspect of the invention. Fig. 20 is a cross-section of Fig. 19 along line a-a. The cut line a-a is in the crotch portion of the diaper 110. The disposable diaper 110 illustrated in Figs. 19 and 20 has a liquid permeable topsheet 111, a liquid impermeable or water repellent backsheet 112, and a central absorbent member 113 disposed between the sheets 111 and 112. The disposable diaper 110 has an oblong, substantially rectangular shape. The liquid permeable topsheet 111 is formed of a sheet material narrower than the backsheet 112. A water repellent nonwoven fabric 114 is interposed between the central absorbent member 113 and the backsheet 112. Each of these elements making the diaper 110 can be of any materials known in the art.

The diaper 110 has a waist flap 117 extending from each of the front side and the rear side ends of the central absorbent member 113. A pair of fastening tapes 118 are attached to both lateral sides of one of the waist flaps 117. The fastening tape 118 has on inner side thereof (the same side as the topsheet side) a fastener for fastening itself to the backsheet 112. Suitable fasteners include a mechanical fastener such as a hook and loop material and a pressure-sensitive adhesive. A waist elastic band 117a stretchable in the diaper width direction is disposed in its stretched state along the edge of the waist flap 117.

The diaper 110 has a pair of leg flaps 115 extending from the lateral side edges of the central absorbent member 113. The leg flaps 115 each have disposed therein leg elastic strands 115a for elastic compliance to the wearer's legs. The leg elastic strands 115a are arranged in both longitudinal side portions of the diaper 110 extending along the length direction of the diaper 110. The leg elastic strands 115a are fixedly held between the backsheet 112 and the water repellent nonwoven fabric 114.

A pair of standing cuffs 116 are formed along the longitudinal sides of the diaper 110 on the topsheet side. The standing cuffs 116 each contain a cuff elastic member 116a along the free edge to create a standing gather.

As illustrated in Fig. 20, the diaper 110 contains an absorbent member 101 according to the second aspect in each of the leg flaps 115 between the water repellent nonwoven fabric 114 and the sheet forming the standing cuff 116. The absorbent member disposed in the leg flap 115 will be referred to as a side absorbent member. The side absorbent member 101 is disposed with the orientation direction of the web (the direction perpendicular to the paper plane of Fig. 20) coinciding with the stretch direction of the leg elastic strands 115a.

Should a body fluid spill over the standing cuff 116, it will be absorbed by the side absorbent member 101 disposed in the leg flap 115. Leakage from the leg opening is thus prevented effectively. Since the orientation direction of the web in the side absorbent member 101 agrees with the length direction of the diaper 110, the liquid absorbed by the side absorbent member 101 distributes preferentially in the length direction of the diaper, which is also effective in preventing leakage from the leg opening. Since the side absorbent member 101 stretches in the same direction as the leg elastic strands 115a laid in the leg flap 115, the leg flaps 115 provide a snug fit to the legs of the wearer, leaving little gap between the leg flaps 115 and the wearer's body. As a result, the leakage from the leg opening is prevented more effectively. In a conventional disposable diaper using a conventional absorbent member made mainly of pulp, more and thicker leg elastic strands should be used to make the absorbent member stretchable. It would follow that an increased amount of an adhesive is required, which results in a sticky feel and reduction in flexibility. Moreover, the conventional absorbent member has to have an increased thickness to secure high absorption capacity, which has resulted in reduction of fit In contrast, using the extensible absorbent member of the present invention eliminates such inconveniences associated with the conventional absorbent member.

A modification of the disposable diaper of Figs. 19 and 20 is represented by Fig. 21, which corresponds to a cross-sectional view taken along line b-b in Fig. 19. The cut line b-b is in the front section of the diaper 110, where leakage from the front side can occur so that high absorptivity is demanded of the absorbent member. The diaper 110 illustrated in Fig. 21 has a hydrophilic web 119 having long fibers disposed between the central absorbent member 113 and the water repellent nonwoven fabric 114. The web 119 interconnects the left and the right side absorbent members 101 to allow for liquid migration therebetween. The long fibers of the web 119 are highly oriented in the width direction of the diaper 100.

The web 119 may have the similar structure as the web 102 of the side absorbent member 101. In some cases, the web 119 does not need to have a superabsorbent polymer embeddedly supported therein, and the long fibers making up the web 119 do not need to have crimp.

According to the modification shown in Fig. 21, if a body fluid runs off the edge of one of the standing cuffs 116 and starts flowing laterally, it is absorbed by the side absorbent member 101 disposed in the leg flap 115, rapidly distributed in the web 119 interconnecting the left and the right hand-sided side absorbent members 101 toward the widthwise middle of the diaper 110, and absorbed by the central absorbent member 113 having high absorption capacity. In brief, the spilt body fluid returns from the side absorbent member 101 to the central absorbent member 113. Part of the fluid diffusing in the web 119 may reach the other side absorbent member 101 and be absorbed there. Such an absorption mechanism allows for effective use of the absorbing performance of the whole absorbent member and secures more effective prevention of leakage.

Another modification of the disposable diaper of Figs. 19 and 20 is given in Fig. 22. Fig. 22 is a cross-sectional view taken along line b-b in Fig. 19 (corresponding to Fig. 20). The diaper illustrated in Fig. 22 has a hydrophilic web 119 having long fibers disposed between the central absorbent member 113 and the water repellent nonwoven fabric 114. The web 119 interconnects the left and the right hand side absorbent members 101 to allow for liquid migration therebetween. The long fibers of the web 119 are highly oriented in the width direction of the diaper 110. The web 119 can be of the same kind as that used in Fig. 21. A liquid impermeable or water repellent sheet 120 is disposed between the web 119 and the central absorbent member 113 to insulate the web 119 from the central absorbent member 113 with regard to liquid migration.

According to the modification shown in Fig. 22, if a body fluid runs off the edge of one of the standing cuffs 116 and starts flowing laterally, it is absorbed by the side absorbent member 101 disposed in the leg flap 115, and rapidly distributed in the web 119 interconnecting the left and the right hand-sided side absorbent members 101 toward the widthwise middle of the diaper 110. Since the web 119 is insulated from the central absorbent member 113, the body fluid migrates in the web 119 and reaches the other side absorbent member 101, where it is absorbed. In short, distribution of liquid between the pair of side absorbent members takes place in the diaper 110. Such liquid distribution also allows for effective use of the absorbing performance of the whole absorbent member. The liquid distribution between the two side absorbent members 101 in Fig. 22 is different from the liquid distribution between the side absorbent members 101 and the central absorbent member 113 in Fig. 21. The structure illustrated in Fig. 21 is designed for the relatively wide front portion of the diaper where high absorption capacity is demanded, whereas that in Fig. 22 is designed for the relatively narrow crotch portion of the diaper where high absorption capacity is not required.

Although the embodiments illustrated in Figs. 19 through 22 present use of the absorbent member according to the second aspect as a side absorbent member in the leg flaps, application of the absorbent member of the second aspect is not limited to that mode of use. For example, the absorbent member of the second aspect may be used as the central absorbent member instead of the side absorbent members, or all the central absorbent member and the two side absorbent members may be the absorbent member of the second aspect in Figs. 19 to 22. The absorbent member of the second aspect may also be disposed in the waist flap 117 with the orientation direction of the web in the absorbent member coinciding with the diaper width direction. The absorbent member thus disposed in the waist flap distributes the absorbed body fluid in the diaper width direction thereby effectively preventing leakage from the waist opening. The absorbent member of the second aspect is also applicable to sanitary napkins or incontinence pads.

In an absorbent article having the absorbent member according to the second aspect, the elastic region around the leg openings and the waist opening may be provided by fixing an elastic member, such as an elastic string, in the stretched state, followed by releasing the elastic member from tension as generally practiced in the art. In addition, there is another method in which an extensible material that can be fixed in its natural state and yet exhibits extensibility. Such an extensible material includes an extensible sheet obtained by uniting a non-extensible nonwoven fabric and an extensible nonwoven fabric and then cutting the non-extensible nonwoven fabric. The latter method is advantageous in that the elastic region hardly leaves indentations or marks on the wearer's skin. When the latter type of an extensible material is combined with a conventional inextensible absorbent member, the extensibility of the extensible material is not manifested. The combined use of such an extensible material with the extensible absorbent member according to the present invention allows for the extensible material exhibiting its extensibility without leaving marks on the skin.

While in the foregoing embodiments the fibrous material of the absorbent member is a web of hydrophilic long fibers, the web may contain other common absorbent fibers such as fluff pulp or synthetic resin staple fibers as long as the extensibility of the absorbent member is not impaired.

The absorbent article having the absorbent member of the second aspect may have not only the liquid permeable sheet 104, in which the web 102 is wrapped, but also the topsheet subjected to any processing for extensibility development. The processing on the liquid permeable sheet and that on the topsheet may be the same or different. For example, uniting the topsheet and the absorbent member may be followed by processing the topsheet to make it extensible. In another approach, the liquid permeable sheet having been processed for extensibility development (e.g., slitting) and the topsheet having been separately processed for extensibility development (e.g., pleating) may be united together. In still another approach, the liquid permeable sheet is subjected to a processing for extensibility development (e.g., slitting), and an extensible topsheet, for example, a perforated film or a perforated nonwoven fabric, is superposed thereon.

In the disposable diaper 110, an embodiment of the third aspect, the central absorbent member 113 may be composed of an airlaid fiber layer made of fluff pulp and a superabsorbent polymer similarly to conventional absorbent members or an extensible absorbent member composed of a hydrophilic crimped long fiber web having a superabsorbent polymer embeddedly supported therein. In the latter case, the absorbent member is preferably disposed with the orientation direction of the web agreeing with the length direction of the disposable diaper 110. The web may be wrapped in a liquid permeable sheet as illustrated in Fig. 15, 17 or 18.

As long as the disposable diaper 110 has the side absorbent member 101 in each leg flap 115, the central absorbent member 113 may be omitted. In such a configuration, an absorbent pad, for example a detachable urine pad may be attached between the leg flaps 115.

While the diaper 110 illustrated in Figs. 19 to 22 has the side absorbent members 101 disposed on the upper side of the leg elastic strands 115a, the side absorbent members may each be disposed on the lower side or by the side of the leg elastic strands 115a.

The absorbent member 101 according to the second aspect can be disposed on other sites than the leg flaps. For instance, it can be disposed m the waist portion or the standing gathers, which are elastic regions extending in the longitudinal or width direction of the diaper located along or outboard of the periphery of the central absorbent member, i.e., lateral sides an/or longitudinal ends of the diaper. Using the absorbent member 101 in the waist portion is effective in preventing leakage from the waist opening, and using in the standing gathers is effective in preventing leakage from the crotch portion. The absorbent member 101 can also be disposed in two or more of the recited sites depending on the intended leak preventive designing. For instance, disposing the absorbent member 101 in the waist portion and the pair of the leg flaps or the pair of the standing gathers is effective in preventing leakage from the waist and the leg openings. The absorbent member 101 disposed in both the pair of the standing gathers and the pair of the leg flaps is more effective in preventing leakage from the crotch portion than when disposed in either one of them.

In the embodiments illustrated in Figs. 21 and 22, the water repellent nonwoven fabric 114 may be removed, in which case the leg elastic strands 115a are fixedly held in between the backsheet 112 and the liquid permeable sheet 104 constituting the absorbent member 101.

The present absorbent members will now be illustrated in greater detail by way of Examples. Unless otherwise noted, all the parts and percents are by weight.

EXAMPLE 1-1

An absorbent member for application to disposable diapers for babies was produced. A tow of crimped long fibers of cellulose acetate was prepared. The long fibers had a diameter of 2.1 dtex. The total diameter of the tow was 25,000 dtex. The tow was opened into an even layer having a width of 100 mm in an air opening apparatus and stretched up to half the maximum length to obtain an opened web having a crimp percentage of 70% and 15 crimps per centimeter. A hot melt adhesive was sprayed at a rate of 5 g/m$^2$ on the upper side of the opened web. Superabsorbent polymer particles were uniformly sprinkled in a layer in an amount of 130 g/m$^2$. After the sprinkling, the opened web was released from the stretched state, whereupon the web contracted to have the superabsorbent polymer particles embeddedly supported therein.

The above operations were conducted once more to prepare a laminate composed of two webs and two polymer layers. A fluff pulp layer weighing 50 g/m$^2$ and a fluff pulp layer weighing 100 g/m$^2$ were put on the upper and the lower sides of the laminate, respectively. The whole structure was wrapped in tissue paper having a grammage of 16 g/m$^2$ to obtain an absorbent member having a weight of 488 g/m$^2$ and a thickness of 2.2 mm. The weight of each of the webs was 13 g/m$^2$.

EXAMPLE 1-2

An absorbent member was made in the same manner as in Example 1-1, except that the hot melt adhesive was not applied between the two webs. The resulting absorbent member had a weight of 443 g/m$^2$ and a thickness of 2.7 mm.

EXAMPLE 1-3

An absorbent member was prepared in the same manner as in Example 1-2, except that each of the webs weighed 60 g per square meter and that no fluff pulp layers was disposed on either side of the laminate. The resulting absorbent member had a weight of 422 g/m$^2$ and a thickness of 1.9 mm.

EXAMPLE 1-4

An opened web having superabsorbent polymer particles embeddedly supported therein was prepared in the same manner as in Example 1-1. The web and the superabsorbent polymer had a weight of 25 g/m$^2$ and 180 g/m$^2$, respectively. Separately, 100 parts of opened fluff pulp and 100 parts of a superabsorbent polymer were uniformly mixed in an air stream and deposited to prepare a pulp/superabsorbent polymer airlaid layer weighing 150 g/m$^2$. The web and the airlaid layer were bonded with 5 g/m$^2$ of a hot melt adhesive, and the resulting laminate was wrapped in tissue paper having a grammage of 16 g/m$^2$ using 5 g/m$^2$ of a hot melt adhesive applied between the tissue paper and the upper and the lower side of the laminate. The resulting absorbent member had a weight of 402 g/m$^2$ and a thickness of 2.0 mm.

EXAMPLE 1-5

A tow of crimped cellulose acetate long fibers was prepared. The long fibers had a diameter of 2.1 dtex. The total diameter of the tow was 25,000 dtex. The tow was opened into an opened web in an air opening apparatus while being carried under tension. The opened web had a crimp percentage of 70% and 15 crimps per centimeter. The opened web was combed by passing between a roll having many discs on its shaft at a prescribed interval and a smooth backup roll. The width of the web was then adjusted to 100 mm. The running speed was reduced, and the web was transferred to a vacuum conveyer, on which the web was relaxed from the tension to make the long fibers to restore the crimp. As a result, the interfiber spaces broadened for helping superabsorbent polymer particles enter, and the web increased in thickness to have improved polymer supporting properties. A superabsorbent polymer was then sprinkled on the web in an amount of 110 g/m$^2$ and embeddedly supported in the web. The web had a basis weight of 26 g/m$^2$.

Separately, 100 parts of opened fluff pulp and 100 parts of a superabsorbent polymer were uniformly mixed in an air stream and deposited to prepare a pulp/superabsorbent polymer airlaid layer having a basis weight of 300 g/m$^2$. The above-prepared web and the airlaid layer were bonded with 5 g/m$^2$ of a hot melt adhesive, and the resulting combined layers were wrapped in tissue paper having a basis weight of 16 g/m$^2$ using 5 g/m$^2$ of a hot melt adhesive applied between the tissue paper and the upper and the lower side of the laminate. The resulting absorbent member had a basis weight of 488 g/m$^2$ and a thickness of 2.1 mm.

COMPARATIVE EXAMPLE 1-1

A hundred parts of opened fluff pulp and 100 parts of a superabsorbent polymer were uniformly mixed in an air stream and deposited to prepare a pulp/superabsorbent polymer airlaid structure having a basis weight of 520 g/m$^2$ (260 g/m$^2$ fluff pulp-260 g/m$^2$ superabsorbent polymer). The airlaid structure was wrapped in tissue paper having a basis weight of 16 g/m$^2$ with 5 g/m$^2$ of a hot melt adhesive applied by spraying to obtain a comparative absorbent member. The resulting absorbent member had a basis weight of 562 g/m$^2$ and a thickness of 4.3 mm.

COMPARATIVE EXAMPLE 1-2

An absorbent member was prepared in the same manner as in Comparative Example 1-1, except that a mixture of fluff pulp and a superabsorbent polymer was airlaid into an airlaid structure having a basis weight of 300 g/m$^2$ (150 g/m$^2$ fluff pulp-150 g/m$^2$ superabsorbent polymer). The resulting absorbent member had a basis weight of 342 g/m$^2$ and a thickness of 2.7 mm.

COMPARATIVE EXAMPLE 1-3

Preparation of an airlaid structure having a basis weight of 375 g/m$^2$ was attempted by using 100 parts of fluff pulp and 200 parts of a superabsorbent polymer in the same manner as in Comparative Example 1-1. However, the attempt was failed because excess of the superabsorbent polymer dropped from the air stream.

Evaluation of performance:

The absorbent members obtained in Examples 1-1 to 1-5 and Comparative Examples 1-1 to 1-3 were measured for absorption capacity and evaluated for structural stability and flexibility in accordance with the methods below. The results obtained are shown in Table 1-1 below.

(1) Absorption capacity

The absorbent member was fixed to an inclined plate set at 45°. A given amount of physiological saline was poured at a given time interval at a position 200 mm downward from the upper end of the absorbent member. The amount of physiological saline that had been poured until it began to leak from the lower end of the absorbent member was taken as an absorption capacity. Taking the absorption capacity of Comparative Example 1-1 as 1.0, the results were expressed relatively by calculation using equation:

$$\text{Absorption capacity (relative)} = \text{absorption capacity of sample/absorption capacity of Comparative Example 1-1}$$

(2) Structural stability

(2-1) While dry

The absorbent member measuring 100 mm by 200 mm was cut into halves along the lateral centerline to make a piece measuring 100 mm by 100 mm. The piece was shaken 20 times at an amplitude of 5 cm at a rate of one shake per second with the cut area down. The polymer particles fallen from the cut area was weighed. The polymer supporting properties, namely, the structural stability of the absorbent member was rated based on the ratio of the fallen polymer to the total polymer as follows.

A: The ratio of the fallen polymer is within 10%.
B: The ratio of the fallen polymer is higher than 10% and not higher than 25%.
C: The ratio of the fallen polymer is higher than 25%.

(2-2) While wet

A 100 mm by 200 mm cut piece of the absorbent member was almost uniformly impregnated with 200 g of physiological saline and then gently lifted to see if the absorbent member suffered from destruction by visual observation.

A: The ratio of the fallen polymer is within 10%. No structural destruction of the absorbent member is observed.
B: The ratio of the fallen polymer is higher than 10% and not higher than 25%. No structural destruction of the absorbent member is observed.
C: The ratio of the fallen polymer is higher than 25%, or structural destruction of the absorbent member is observed.

(3) Flexibility

Flexibility of the absorbent member was evaluated by the use of a handle-o-meter HOM-3 available from Daiei Kagaku Seiki Co., Ltd. The smaller the "handle" value as measured with a handle-o-meter, the easier to put on and the snugger the fit. Measurement was carried out as follows in accordance with JIS L 1096 (stiffness and softness measuring method). A specimen measuring 50 mm wide and 150 mm long was placed on the platform having a 60 mm wide slot with the length perpendicular to the slot. The force required for a 2 mm thick penetrator blade to force the center of the specimen into the slot was read. The measurement was made on three points to obtain an average. The flexibility was rated as follows.

A: The "handle" is 2N or less.
B: The "handle" is more than 2N and not more than 4N.
C: The "handle" is more than 4N.

TABLE 1-1

| | | Example | | | | | Comp. Example | | |
|---|---|---|---|---|---|---|---|---|---|
| | | 1-1 | 1-2 | 1-3 | 1-4 | 1-5 | 1-1 | 1-2 | 1-3 |
| Thickness (mm) | | 2.2 | 2.7 | 1.9 | 2.0 | 2.1 | 4.5 | 2.7 | - |
| Amount of Long Fiber (g/m$^2$) of Long Fiber | | 26 | 26 | 120 | 25 | 26 | 0 | 0 | 0 |
| Amount of Pulp (g/m$^2$) | | 150 | 150 | 0 | 75 | 150 | 260 | 150 | 125 |
| Amount of Superabsorbent Polymer (g/m$^2$) | | 260 | 260 | 260 | 255 | 260 | 260 | 150 | 250 |
| Absorption Capacity | | 1.3 | 1.3 | 1.0 | 1.3 | 1.3 | 1.0 | 0.6 | - |
| Structural Stability | Dry | A | A | A | A | A | A | A | C |
| | Wet | A | A | A | A | A | A | C | - |

(continued)

|  | Example | | | | | Comp. Example | | |
| --- | --- | --- | --- | --- | --- | --- | --- | --- |
|  | 1-1 | 1-2 | 1-3 | 1-4 | 1-5 | 1-1 | 1-2 | 1-3 |
| Flexibility | A | A | A | A | A | C | A | - |
| Note: In Comparative Example 1-3, not all the measurement and evaluation were carried out because of failure to obtain an intended absorbent member. | | | | | | | | |

It is clear from Table 1 that the absorbent members of Examples exhibit structural stability notwithstanding a smaller proportion of the fibrous materials than the comparative ones. It is also seen that the absorbent members of Examples are so light weight and flexible and yet highly absorptive.

EXAMPLE 1-6

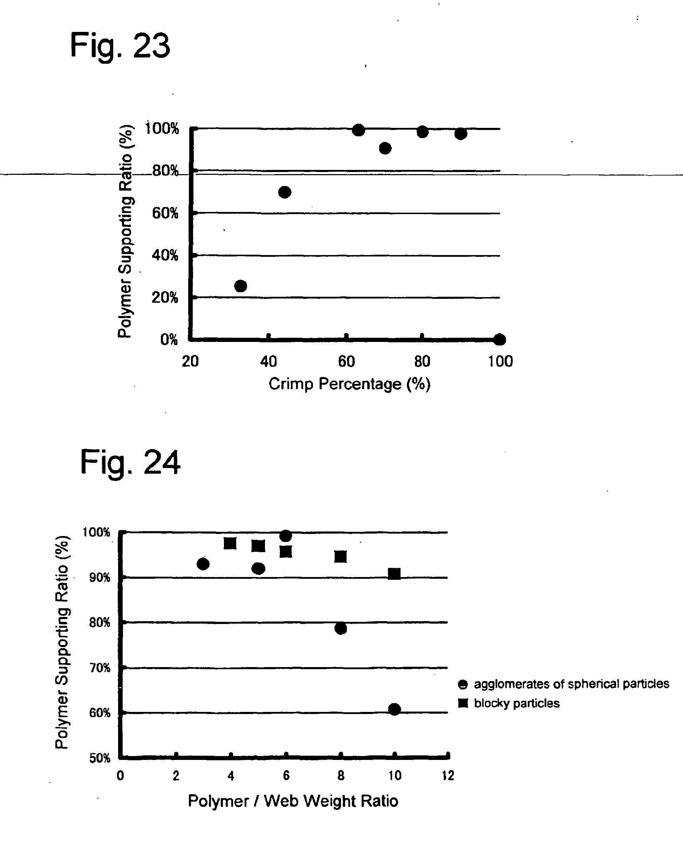

The relation between crimp percentage of a long fiber web and ability of the web to support superabsorbent polymer (polymer supporting ratio) was examined. Tows of cellulose acetate long fibers having a fiber diameter of 2.1 dtex were prepared. Each of the tow was carried under tension and opened in an air opening apparatus to obtain an opened web. The opened web was combed through a roll having many discs on its shaft at a prescribed interval and a smooth backup roll. The width of the web was then adjusted to 100 mm. The running speed was reduced, and the web was transferred to a vacuum conveyer, on which the web was relaxed from the tension to make the long fibers to restore the crimp. The crimp percentage of the web was varied by controlling the tension of the web. As a result, the interfiber spaces broadened for helping superabsorbent polymer particles enter, and the web increased in thickness to have improved properties of embeddedly supporting superabsorbent polymer particles. Blocky superabsorbent polymer particles having a mean particle size of 330 $\mu$m were then sprinkled on the web in an amount of 260 g/m$^2$ and embeddedly supported in the web. The web had a weight of 26 g/m$^2$. The absorbent member thus prepared was tested for structural stability (while dry) in according with the method described above. After the test, the weight of the polymer remaining supported in the web was divided by the weight of the web, and the quotient was multiplied by 100 to give a polymer supporting ratio (%). The results obtained are graphically represented in Fig. 23. As is apparent from Fig. 23, long fiber webs having a crimp percentage ranging from 40% to 90% have a high polymer supporting ratio

EXAMPLE 1-7

The relation between particle shape of superabsorbent polymer and supportability of the particles in a web was examined. Tows of cellulose acetate crimped long fibers having a fiber diameter of 2.1 dtex were prepared. In the same manner as in Example 1-6, each tow was opened to obtain a web having a crimp percentage of 70%, and superabsorbent polymer particles were sprinkled on the web in a varied amount per unit area and embeddedly supported in the web. The web had a basis weight of 26 g/m$^2$. Blocky superabsorbent polymer particles or agglomerates of spherical superabsorbent polymer particles were used. The blocky superabsorbent polymer particles had a mean particle size of 330 $\mu$m. The agglomerates of spherical superabsorbent polymer particles had a mean particle size of 400 $\mu$m. The absorbent member thus prepared was tested for structural stability (while dry) to obtain a polymer supporting ratio (%) in the same manner as in Example 1-6. The results obtained are graphically represented in Fig. 24. The graph of Fig. 24 proves the blocky particles more easily supportable than agglomerates of spherical particles.

EXAMPLE 1-8

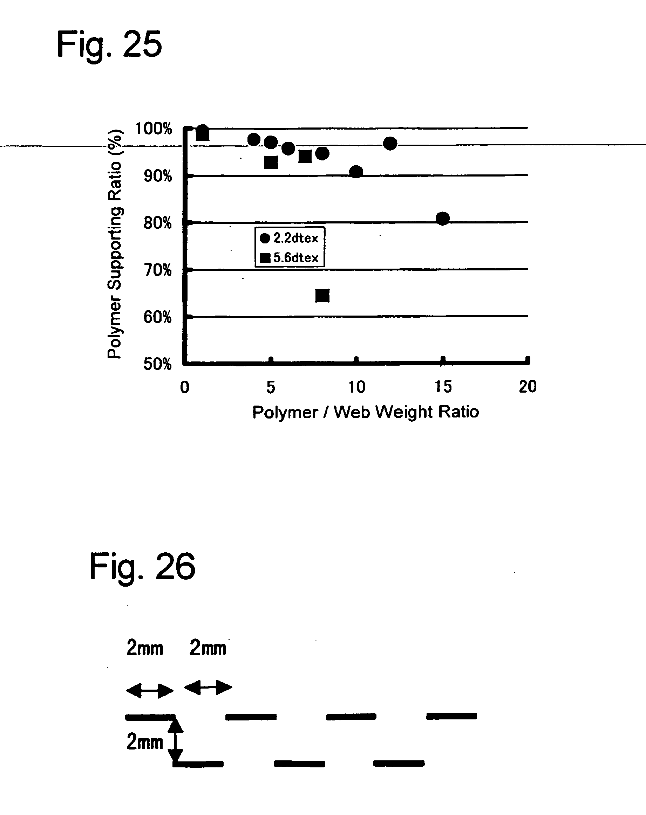

The relation between fiber diameter and polymer supporting properties of a long fiber web was examined. Two kinds of tows of cellulose acetate long fibers having a fiber diameter of 2.2 dtex and 5.6 dtex were prepared. In the same manner as in Example 1-6, each tow was opened to obtain a web having a crimp percentage of 70%. Blocky superabsorbent polymer particles having a mean particle size of 330 $\mu$m were sprinkled on the web in a varied amount per unit area and embeddedly supported in the web. The web had a weight of 26 g/m$^2$. The absorbent member thus prepared was tested for structural stability (while dry) to obtain a polymer supporting ratio (%) in the same manner as in Example 1-6. The results obtained are graphically represented in Fig. 25. The graph of Fig. 25 proves that the webs of long fibers with a diameter of 2.2 dtex and 5.6 dtex both exhibit satisfactory polymer supporting properties.

Mean particle size of the superabsorbent polymer was measured as follows. A superabsorbent polymer (50g) was sieved by sieve shaker AS200 available from Retsch. Sieves were available from Tokyo Screen. The sieves had a mesh size, in accordance with JIS Z8801, of 850, 600, 500, 355, 300, 250 and 150, respectively. The sieving was carried out at a frequency of 50 Hz, an amplitude of 0.5 mm for 10 minutes. Measurement was carried out for three times. The average weight of the polymer which remained on the sieve was calculated. The average weight was divided by 50 to obtain a relative frequency. Then, a cumulative particle size curve was obtained based on the relative frequency. Mean particle size was defined as a particle size which corresponds to the cumulative half value (50%) in the cumulative particle size curve.

EXAMPLE 2-1

A web of crimped long fibers of cellulose acetate was prepared. The long fibers had a diameter of 2.1 dtex. The total diameter of the web was 25,000 dtex. The web was opened into an even layer having a width of 100 mm in an air opening apparatus and stretched up to the maximum length to obtain an opened web. A hot melt adhesive was sprayed at a rate of 5 g/m$^2$ on the upper side of the opened web. Superabsorbent polymer particles were uniformly sprinkled in a layer in an amount of 130 g/m$^2$. After the sprinkling, the opened web was freed from the stretched state to obtain the web having a crimp percentage of 70% and 15 crimps per centimeter, whereupon the web contracted to have the superabsorbent polymer particles embeddedly supported therein to obtain a first laminate. The above operations were conducted once more to prepare a second laminate. The first laminate was superposed on the second laminate with a fluff pulp layer weighing 50 g/m$^2$ interposed therebetween.

A fluff pulp layer having a weight of 100 g/m$^2$ was placed beneath the second laminate. The whole structure was wrapped in tissue paper having a grammage of 16 g/m$^2$ on which a hot melt adhesive had been sprayed to obtain a central absorbent member. The central absorbent member was made extensible as a whole by making slits each extending in the direction perpendicular to the orientation direction of the webs in a pattern illustrated in Fig. 26, in which slits each having a length of 2 mm were arranged in the width direction at a distance of 2 mm and alternately arranged in the length direction at a distance of 2 mm. The central absorbent member was used with the fluff pulp layer of 100 g/m$^2$ on the garment facing side. The central absorbent member had a weight of 488 g/m$^2$ and a thickness of 2.2 mm. The weight of each of the webs was 13 g/m$^2$.

A disposable diaper was fabricated using the resulting central absorbent member with the orientation direction of the webs coinciding with the diaper length direction in a usual manner. The topsheet was an air-through nonwoven fabric weighing 25 g/m$^2$, made of linear low density polyethylene sheath/polypropylene core conjugate fiber (diameter: 2.1 dtex; having been treated with surface active agent to have liquid permeability), and having been perforated with 5 mm diameter metal pins. The backsheet was a laminate of porous film having a grammage of 20 g/m$^2$ and spun-bonded polypropylene nonwoven fabric having a weight of 20 g/m$^2$, bonded with 1.5 g/m$^2$ of a hot melt adhesive. The porous film was a product produced by blown-film extruding a uniform mixture of 100 parts of linear low density polyethylene (density: 0.925 g/cm$^2$), 150 parts of calcium carbonate, and 4 parts of an ester compound and longitudinally stretching to double the length.

EXAMPLE 2-2

A disposable diaper was obtained in the same manner as in Example 2-1, except that any fluff pulp layer was not disposed in the absorbent member, each web had a weight of 60 g/m$^2$, and the tissue paper for wrapping the laminate was replaced with a hydrophilized spunbond-meltblown-spunbond (SMS) nonwoven fabric weighing 16 g/m$^2$ and having been subjected to slitting to have slits cut in the same pattern as in Example 2-1.

EXAMPLE 2-3

An absorbent member was obtained in the same manner as in Example 2-1 with the following exceptions. Any fluff pulp layer was not disposed in the absorbent member. Each web had a weight of 60 g/m$^2$. The tissue paper for wrapping the laminate was replaced with a hydrophilized SMS nonwoven fabric having a weight of 16 g/m$^2$. Wrapping of the laminate structure in the SMS nonwoven fabric was carried out while the laminate was in the stretched state. A disposable diaper was fabricated using the resulting absorbent member in the same manner as in Example 2-2.

EXAMPLE 2-4

A side absorbent member was produced as follows. A web of crimped long fibers of cellulose acetate was prepared. The long fibers had a diameter of 2.1 dtex. The total diameter of the web was 25,000 dtex. The web was opened into an even layer having a width of 50 mm in an air opening apparatus and stretched up to the maximum length. A hot melt adhesive was sprayed at a rate of 5 g/m$^2$ on the upper side of the opened web. Superabsorbent polymer particles were uniformly sprinkled in a layer in an amount of 150 g/m$^2$. After the sprinkling, another, separately prepared opened web was superposed thereon, and the opened webs were freed from the stretched state to obtain the webs having a crimp percentage of 70% and 15 crimps per centimeter, whereupon the webs contracted to have the superabsorbent polymer particles embeddedly supported therein. The resulting laminate was wrapped in a hydrophilized SMS nonwoven fabric having a weight of 16 g/m$^2$ and having been slitted in the same manner as in Example 2-2. The side absorbent member thus obtained had a weight of 173 g/m$^2$ and a diameter of 1.0 mm, in which each web had a weight of 13 g/m$^2$. A disposable diaper illustrated in Fig. 19 was fabricated in the same manner as in Example 2-1, except that the side absorbent member prepared above was disposed in the leg flaps with the orientation direction of the webs coinciding with the diaper length direction.

COMPARATIVE EXAMPLE 2-1

A hundred parts of opened fluff pulp and 100 parts of a superabsorbent polymer were uniformly mixed in an air stream and deposited to prepare a pulp/superabsorbent polymer airlaid structure having a weight of 520 g/m$^2$ (260 g/m$^2$ fluff pulp and 260 g/m$^2$ superabsorbent polymer). The airlaid structure was wrapped in tissue paper having a grammage of 16 g/m$^2$ with 5 g/m$^2$ of a hot melt adhesive applied by spraying to obtain an absorbent member. The resulting absorbent member had a weight of 562 g/m$^2$ and a thickness of 4.3 mm. A disposable diaper was fabricated in the same manner as in Example 2-1, except for using the resulting absorbent member.

COMPARATIVE EXAMPLE 2-2

A side absorbent member was prepared as follows. A hundred parts of opened fluff pulp and 100 parts of a superabsorbent polymer were uniformly mixed in an air stream and deposited to prepare a pulp/superabsorbent polymer airlaid structure having a weight of 300 g/m$^2$ (150 g/m$^2$ fluff pulp and 150 g/m$^2$ superabsorbent polymer). The airlaid structure was wrapped in tissue paper having a grammage of 16 g/m$^2$ with 5 g/m$^2$ of a hot melt adhesive applied by spraying to obtain a side absorbent member. The resulting side absorbent member had a weight of 342 g/m$^2$ and a thickness of 2.6 mm. A disposable diaper was fabricated in the same manner as in Comparative Example 2-1, except for disposing the resulting side absorbent member in the leg flaps.

Evaluation of performance:

The absorbent members obtained in Examples 2-1 to 2-4 and Comparative Examples 2-1 to 2-2 were evaluated for absorption capacity, flexibility, and structural stability in accordance with the methods described below. The disposable diapers obtained were evaluated for fit. The results are shown in Table 2-1 below.

(1) Absorption capacity

The absorbent member was fixed to an inclined plate set at 45°. A given amount of physiological saline was poured at a given time interval at a position 200 mm downward from the upper end of the absorbent member. The amount of saline that had been poured until it began to leak from the lower end of the absorbent member was taken as an absorption capacity. Taking the absorption capacity of Comparative Example 2-1 as 1.0, the results were expressed relatively by calculation using equation:

$$\text{Absorption capacity (relative)} = \text{absorption capacity of sample/absorption capacity of Comparative Example 2-1}$$

(2) Flexibility

Flexibility of the absorbent member was evaluated by handle-o-meter test. The smaller the "handle" value as measured with a handle-o-meter, the easier to put on and the snugger the fit. The flexibility was rated as follows.

A: The "handle" is 2N or less.
B: The "handle" is more than 2N and not more than 4N.
C: The "handle" is more than 4N.

(3) Structural stability

(3-1) While dry

The absorbent member measuring 50 mm by 200 mm was cut into halves along the lateral centerline to make a piece measuring 50 mm by 100 mm. The piece was shaken 20 times at an amplitude of 5 cm at a rate of one shake per second with the cut area down. The polymer particles fallen from the cut area was weighed. The polymer supporting properties, namely, the structural stability of the absorbent member was rated based on the ratio of the fallen polymer to the total polymer as follows.

A: The ratio of the fallen polymer is within 10%.
B: The ratio of the fallen polymer is higher than 10% and not higher than 25%.
C: The ratio of the fallen polymer is higher than 25%.

(3-2) While wet

A 50 mm by 200 mm cut piece of the absorbent member was almost uniformly impregnated with 100 g of physiological saline and then gently lifted to see if the absorbent member suffered from destruction by visual observation.

A: The ratio of the fallen polymer is within 10%. No structural destruction of the absorbent member is observed.
B: The ratio of the fallen polymer is higher than 10% and not higher than 25%. No structural destruction of the absorbent member is observed.
C: The ratio of the fallen polymer is higher than 25%, or structural destruction of the absorbent member is observed.

(4) Fit

The disposable diapers prepared were worn by five babies of 7 to 14 months of age who usually wore medium size diapers. The baby's mothers were asked to rate the diapers in terms of fit in accordance with the following rating system.

A: A snug fit is provided with no bagginess around the crotch.
B: A slightly snug fit is provided.
C: A snug fit is not provided. Bagginess around the crotch is felt.

TABLE 2-1

| | | Example | | | | Comp. Example | |
|---|---|---|---|---|---|---|---|
| | | 2-1 | 2-2 | 2-3 | 2-4 | 2-1 | 2-2 |
| Central Absorbent Member | Material | long fiber web | long fiber web | long fiber web | long fiber web | pulp/ polymer | pulp/ polymer |
| | Wrapping Sheet | tissue paper with slits | hydrophilized SMS with slits | hydrophilized SMS with slits | tissue paper | tissue paper | tissue paper |
| | Thickness (mm) | 2.2 | 2.0 | 3.0 | 2.0 | 4.5 | 4.5 |
| Side Absorbent Member | Material | - | - | - | long fiber web | - | pulp/ polymer |
| | Wrapping Sheet | - | - | - | hydrophilized SMS with slits | - | tissue paper |
| | Thickness (mm) | - | - | - | 1.0 | - | 2.7 |

(continued)

| | | Example | | | | Comp. Example | |
|---|---|---|---|---|---|---|---|
| | | 2-1 | 2-2 | 2-3 | 2-4 | 2-1 | 2-2 |
| Absorption Capacity | Central Absorbent Member | 1.0 | 1.3 | 1.3 | 1.0 | 1.0 | 1.0 |
| | Side Absorbent Member | - | - | - | 0.6 | - | 0.6 |
| Flexibility | | A | A | A | A | C | C |
| Structural Stability | Dry | A | A | A | A | A | A |
| | Wet | A | A | A | A | A | C |
| Fit | | A | A | A | A | C | C |

EXAMPLE 3-1

A side absorbent member was made in the same manner as in Example 2-4. A disposable diaper of Fig. 19 was fabricated using the resulting side absorbent member in the leg flaps in a usual manner. The topsheet was an air-through nonwoven fabric weighing 25 g/m$^2$, made of linear low density polyethylene sheath/polypropylene core conjugate fiber (thickness: 2.1 dtex; having been treated with surface active agent to have liquid permeability) and having been perforated with 5 mm diameter metal pins. The backsheet was a laminate of porous film having a grammage of 20 g/m$^2$ and spun-bonded polypropylene nonwoven fabric having a weight of 20 g/m$^2$, bonded together with 1.5 g/m$^2$ of a hot melt adhesive. The porous film was a product produced by blown-film extruding a uniform mixture of 100 parts of linear low density polyethylene (density: 0.925 g/cm$^2$), 150 parts of calcium carbonate, and 4 parts of an ester compound and longitudinally stretching to double the length. The central adsorbent member was an airlaid layer formed by depositing a 1:1 (by weight) mixture of opened fluff pulp and a superabsorbent polymer and wrapped in tissue paper having a grammage of 16 g/m$^2$. The airlaid layer contained 260 g/m$^2$ of the fluff pulp and 260 g/m$^2$ of the superabsorbent polymer.

EXAMPLE 3-2

A web of crimped long fibers of cellulose acetate was prepared. The long fibers had a diameter of 2.1 dtex. The total thickness of the web was 25,000 dtex. The web was opened into an even layer having a width of 50 mm in an air opening apparatus and stretched up to the maximum length. A hot melt adhesive was sprayed at a rate of 5 g/m$^2$ on the upper side of the stretched opened web. Superabsorbent polymer particles were uniformly sprinkled in a layer in an amount of 100 g/m$^2$. After the sprinkling, another, separately prepared opened web was superposed thereon. While the laminate was in the stretched state, it was wrapped in a hydrophilized SMS nonwoven fabric weighing 16 g/m$^2$ and then released from the stretched state. Whereupon, the webs contracted to have the superabsorbent polymer particles embeddedly supported therein. The long fibers had a crimp percentage of 70% and 15 crimps per centimeter. The whole structure was then wrapped in a hydrophilized and slitted SMS nonwoven fabric having a weight of 16 g/m$^2$ to obtain a side absorbent member. The slit pattern of the SMS nonwoven fabric was the same as in Example 2-1. A disposable diaper was fabricated in the same manner as in Example 3-1, except for using the side absorbent member thus prepared.

EXAMPLE 3-3

A disposable diaper was fabricated in the same manner as in Example 3-1, except that the side absorbent member was disposed in the front and the rear waist portions as well as the leg flaps.

COMPARATIVE EXAMPLE 3-1

A disposable diaper was fabricated in the same manner as in Example 3-1, except that the side absorbent member was produced as follows. A hundred parts of opened fluff pulp and 100 parts of a superabsorbent polymer were uniformly mixed in an air stream and deposited to prepare a pulp/superabsorbent polymer airlaid layer having a weight of 200 g/m$^2$. The airlaid layer contained 100 g/m$^2$ of the fluff pulp and 100 g/m$^2$ of the superabsorbent polymer. The airlaid layer was wrapped in tissue paper having a grammage of 16 g/m$^2$ with 5 g/m$^2$ of a hot melt adhesive applied by spraying to obtain a side absorbent member. The resulting side absorbent member had a weight 242 g/m$^2$ and a thickness of 2.1 mm.

Evaluation of performance:

The absorbent members obtained in Examples 3-1 to 3-3 and Comparative Example 3-1 were evaluated for flexibility and structural stability in the same manner as in Example 2-1. The diapers obtained were evaluated for fit in the same manner as in Example 2-1. The results are shown in Table 3-1 below.

TABLE 3-1

| | | Example | | | Comp. Example 3-1 |
|---|---|---|---|---|---|
| | | 3-1 | 3-2 | 3-3 | |
| Side Absorbent Member | Material | long fiber web | long fiber web | long fiber web | pulp/polymer |
| | Wrapping Sheet | hydrophilized SMS | hydrophilized SMS with slits | hydrophilized SMS with slits | Tissue |
| | Site of Use | leg flaps | leg flaps | leg flaps/ waist portions | leg flaps |
| Central Adsorbent Member | Material | pulp/polymer | pulp/polymer | pulp/polymer | pulp/polymer |
| | Wrapping Sheet | tissue | tissue | tissue | tissue |
| Flexibility | | A | A | A | B |
| Structural Stability | Dry | A | A | A | B |
| | Wet | A | A | A | C |
| Fit | | A | A | A | B |

As is apparent from the results in Table 3-1, the absorbent members of Examples are structurally more stable than the comparative one notwithstanding the smaller proportion of fibrous materials. The disposable diapers using the absorbent members of Examples are flexible and excellent in ease of diapering and fit to a baby's body.

As described above, the present invention provides a process of producing an absorbent member for an absorbent article with a smaller thickness and a lighter weight than conventional absorbent members while retaining equal absorption capacity. The absorbent member maintains its structure against wearer's active movement.

The present invention provides an extensible absorbent member. The absorbent article having the extensible absorbent member, particularly in its elastic regions along the lateral sides and/or the front and the rear waist ends, ensures an improved fit to a wearer's body. While worn, the absorbent article is sufficiently conformable to the body's shape changing with movement. Since the absorbent member is deformable according to the wearer's movement, there is less bunching and roping. As a result, a gap is hardly created between the absorbent article and the wearer's body, so that leakage is prevented effectively. Since the absorbent member contains a web, it is more flexible and thinner than the one made mainly of fluff pulp and having equal absorption capacity. Accordingly, the absorbent article having the extensible absorbent member is easy to put on and hardly causes a wearer discomfort while worn.

**Claims**

1. A process of producing an absorbent member comprising a web of crimped long fibers and a superabsorbent polymer contained in the web, the process comprising the steps of opening the web in a longitudinally stretched state under tension and feeding the superabsorbent polymer to the web in a state under reduced tension.

2. The process according to claim 1, wherein an adhesive is applied to the web having the long fibers stretched prior to the step of sprinkling the superabsorbent polymer on said web.

3. The process according to claim 2, wherein the superabsorbent polymer is sprinkled on the web and then the long fibers are released from the stretched state.

**4.** The process according to claims 2 and 3, wherein the step of applying the adhesive is carried out by a non-contact type application system.

**5.** The process according to claim 4, wherein the adhesive is applied by spray coating.


**Patentansprüche**

**1.** Ein Verfahren zur Herstellung eines absorbierenden Elements, umfassend eine Bahn aus gekräuselten langen Fasern und ein in der Bahn enthaltendes superabsorbierendes Polymer, wobei das Verfahren die Schritte des Öffnens der Bahn unter Spannung in einem der Länge nach gestreckten Zustand und des Zuführens des superabsorbierenden Polymers in die Bahn unter einem Zustand verringerter Spannung umfasst.

**2.** Das Verfahren gemäß Anspruch 1, wobei ein Haftmittel auf die Bahn mit den gestreckten langen Fasern vor dem Schritt des Sprenkelns des superabsorbierenden Polymers auf die Bahn aufgebracht wird.

**3.** Das Verfahren gemäß Anspruch 2, wobei das superabsorbierende Polymer auf die Bahn gestreut wird und anschließend die langen Fasern aus ihrem gestreckten Zustand gelöst werden.

**4.** Das Verfahren gemäß den Ansprüchen 2 und 3, wobei der Schritt des Aufbringens des Haftmittels durch ein Aufbringungssystem vom Kontaktfrei-Typ durchgeführt wird.

**5.** Das Verfahren gemäß Anspruch 4, wobei das Haftmittel durch Sprühbeschichtung aufgebracht wird.


**Revendications**

**1.** Procédé pour la production d'un élément absorbant comprenant un tissu de longues fibres plissées et un polymère superabsorbant contenu dans le tissu, le procédé comprenant les étapes consistant à ouvrir le tissu dans un état étiré longitudinalement en exerçant une tension et à apporter le polymère superabsorbant au tissu dans un état sous tension réduite.

**2.** Procédé selon la revendication 1, dans lequel un adhésif est appliqué au tissu ayant les longues fibres étirées préalablement à l'étape consistant à déposer le polymère superabsorbant sur ledit tissu.

**3.** Procédé selon la revendication 2, dans lequel le polymère superabsorbant est déposé sur le tissu et ensuite les longues fibres sont relâchées depuis l'état étiré.

**4.** Procédé selon les revendications 2 et 3, dans lequel l'étape consistant à appliquer l'adhésif est réalisée par un système d'application de type sans contact.

**5.** Procédé selon la revendication 4, dans lequel l'adhésif est appliqué par pulvérisation.

# Fig. 1

# Fig. 2(a)

# Fig. 2(b)

# Fig. 3

# Fig. 4

Fig. 5

Fig. 6(a)

Fig. 6(b)

Fig. 6(c)

Fig. 7(a)

Fig. 7(b)

Fig. 7(c)

Fig. 7(d)

Fig. 8(a)

Fig. 8(b)

Fig. 9

Fig. 10

## Fig. 11

## Fig. 12

## Fig. 13

Fig. 14

Fig. 15

## Fig. 16

## Fig. 17

## Fig. 18

## Fig. 19

EP 1 627 618 B1

Fig. 20

115    116 116a        110    116a 116    115
      101                                  101
              111 113

115a    112    114    115a

Fig. 21

115    116 116a        110    116a 116    115
      101                                  101
              111 113

115a    112    114 119 115a

Fig. 22

115    116 116a        110    116a 116    115
      101                                  101
              111 113

115a 120  112  114 119 115a

37

## Fig. 23

## Fig. 24

● agglomerates of spherical particles
■ blocky particles

# Fig. 25

# Fig. 26

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 57160457 A
- WO 200134082 A
- JP 2001276125 A

- JP 2004159786 A
- JP 7184956 A